(19) Europäisches Patentamt / European Patent Office / Office européen des brevets

(11) **EP 4 282 826 A1**

(12) **EUROPEAN PATENT APPLICATION**
published in accordance with Art. 153(4) EPC

(43) Date of publication:
**29.11.2023  Bulletin 2023/48**

(21) Application number: **22742613.7**

(22) Date of filing: **19.01.2022**

(51) International Patent Classification (IPC):
*C01G 25/02* (2006.01)   *A61C 5/70* (2017.01)
*A61C 13/083* (2006.01)   *A61K 6/818* (2020.01)
*A61K 6/822* (2020.01)   *A61K 6/824* (2020.01)
*C01G 25/00* (2006.01)   *C04B 35/486* (2006.01)
*C04B 35/488* (2006.01)

(52) Cooperative Patent Classification (CPC):
**A61C 5/70; A61C 13/00; A61C 13/083;
A61K 6/818; A61K 6/822; A61K 6/824;
C01G 25/00; C01G 25/02; C04B 35/486;
C04B 35/488**

(86) International application number:
**PCT/JP2022/001817**

(87) International publication number:
**WO 2022/158491 (28.07.2022 Gazette 2022/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **22.01.2021  JP 2021009019
10.11.2021  JP 2021183423**

(71) Applicant: **Tosoh Corporation
Yamaguchi 746-8501 (JP)**

(72) Inventors:
• **AZECHI Sho
Shunan-shi Yamaguchi 746-8501 (JP)**

• **USHIO Yuki
Shunan-shi Yamaguchi 746-8501 (JP)**
• **SHIMIZU Takahiro
Shunan-shi Yamaguchi 746-8501 (JP)**
• **HIGUCHI Yuya
Shunan-shi Yamaguchi 746-8501 (JP)**
• **NAGAYAMA Hitoshi
Shunan-shi Yamaguchi 746-8501 (JP)**
• **FUJISAKI Hiroyuki
Shunan-shi Yamaguchi 746-8501 (JP)**
• **IMAI Kenji
Shunan-shi Yamaguchi 746-8501 (JP)**
• **KAWASHIMA Risa
Shunan-shi Yamaguchi 746-8501 (JP)**

(74) Representative: **Müller-Boré & Partner
Patentanwälte PartG mbB
Friedenheimer Brücke 21
80639 München (DE)**

(54) **POWDER COMPOSITION**

(57)     To provide at least one of: a powder composition from which a calcined body with similar processability can be produced without requiring the application of different forming conditions and calcination conditions for each composition; a method for producing the powder composition; a calcined body produced from the powder composition; a method for producing the calcined body; and uses thereof.

A powder composition comprising: two or more types of zirconia in which a lanthanoid rare-earth element is dissolved; a transition metal element other than zirconium and hafnium; and a remainder composed of zirconia stabilized only by one or more selected from the group consisting of yttrium, calcium and magnesium, wherein a different lanthanoid rare-earth element is dissolved in each zirconia in which the lanthanoid rare-earth element is dissolved, and a transition metal element content is 1500 ppm or less.

EP 4 282 826 A1

**Description**

Technical Field

The present disclosure relates to a powder composition composed mainly of zirconia and a use of the powder composition.

Background Art

Zirconia is applied to dental prostheses, such as crowns and bridges, due to its mechanical characteristics and high esthetics based on transparency/translucency. A dental prosthesis is produced with a CAD/CAM apparatus by grinding a calcined body (also referred to as a semi-sintered body, a presintered body or a blank), which is produced by calcining a green body (green compact) of zirconia. Thus, the calcined body is required to have mechanical characteristics suitable for cutting. For example, Patent Literature 1 discloses a Vickers hardness in the range of 25 to 150 as a mechanical characteristic suitable for cutting.

Also, as indicated by a dental color chart (for example, the Vita classical shade guide), the color tone of natural teeth varies from patient to patient and from tooth type to tooth type. To provide a dental prostheses with a color tone similar to the color tone of a natural tooth, a precolored calcined body is utilized. A colored calcined body is typically produced by a method of calcining a green body of a composition containing a pigment and zirconia homogeneously mixed (what is called a powder mixing method; for example, Patent Literature 1 and Patent Literature 2). In the powder mixing method, the type and amount of pigment are changed to adjust the composition for a desired color tone, thereby preparing a powder composition containing the pigment and a zirconia powder homogeneously mixed. The powder composition is formed and calcined to prepare a uniformly colored calcined body.

Citation List

Patent Literature

    PTL 1: International Publication No. 2014/022643
    PTL 2: U.S. Patent No. 9962247
    PTL 3: International Publication No. 2016/019114

Summary of Invention

Technical Problem

Calcined bodies with different compositions have not only different color tones but also different processability. On the other hand, calcined bodies are cut under the same conditions regardless of the compositions thereof. Thus, in the powder mixing method, different production conditions (forming conditions and calcination conditions) are applied to different compositions to reduce variations in processability due to compositional differences. Due to the necessity for different production conditions, the productivity of a calcined body varies greatly from composition to composition.

It is an object of the present disclosure to provide at least one of: a powder composition from which a calcined body with similar processability can be produced without requiring the application of different forming conditions and calcination conditions for each composition; a method for producing the powder composition; a calcined body produced from the powder composition; a method for producing the calcined body; and uses thereof. Preferably, it is another object to provide at least one of: a method for producing a calcined body capable of reducing variations in productivity; a calcined body produced by the method; and uses thereof.

Solution to Problem

The present invention is as described in the claims, and the gist of the present disclosure is as follows:

[1] A powder composition comprising: two or more types of zirconia in which a lanthanoid rare-earth element is dissolved; a transition metal element other than zirconium and hafnium; and a remainder composed of zirconia stabilized only by one or more selected from the group consisting of yttrium, calcium and magnesium, wherein a different lanthanoid rare-earth element is dissolved in each zirconia in which the lanthanoid rare-earth element is dissolved, and a transition metal element content is 1500 ppm or less.

[2] The powder composition according to [1], wherein at least one type of the zirconia in which the lanthanoid rare-earth element is dissolved is zirconia in which one or more selected from the group consisting of praseodymium, samarium, terbium, dysprosium, holmium and thulium are dissolved.

[3] The powder composition according to [1] or [2], wherein at least one type of the zirconia in which the lanthanoid rare-earth element is dissolved is zirconia in which one or more selected from the group consisting of neodymium and erbium are dissolved.

[4] The powder composition according to any one of [1] to [3], wherein at least one type of the zirconia in which the lanthanoid rare-earth element is dissolved is zirconia stabilized by one or more selected from the group consisting of yttrium, calcium and magnesium.

[5] The powder composition according to any one of [1] to [4], wherein the transition metal element is one or more selected from the group consisting of manganese, cobalt and titanium.

[6] The powder composition according to any one of [1] to [5], wherein the transition metal element is contained as one or more selected from the group consisting of oxides, hydroxides, oxyhydroxides, chlorides, sulfates and nitrates.

[7] The powder composition according to any one of [1] to [6], wherein the remainder is zirconia stabilized only by yttrium.

[8] The powder composition according to any one of [1] to [7], wherein an iron content is 100 ppm or less.

[9] The powder composition according to any one of [1] to [8], comprising alumina.

[10] The powder composition according to any one of [1] to [9], comprising a granular particle composed of the transition metal element other than zirconium and hafnium and the zirconia stabilized only by one or more selected from the group consisting of yttrium, calcium and magnesium.

[11] The powder composition according to any one of [1] to [10], wherein a BET specific surface area is 5 $m^2$/g or more and 15 $m^2$/g or less.

[12] A method for producing a calcined body using the powder composition according to any one of [1] to [11].

[13] A method for producing a sintered body using the powder composition according to any one of [1] to [11].

[14] A calcined body comprising a fused particle of a transition metal compound other than zirconium and hafnium, zirconia in which two or more lanthanoid rare-earth elements are dissolved, and zirconia stabilized only by one or more selected from the group consisting of yttrium, calcium and magnesium, wherein the transition metal element other than zirconium and hafnium constitutes 1500 ppm or less.

[15] A method for producing a sintered body, comprising using the calcined body according to [14].

Advantageous Effects of Invention

The present disclosure can provide at least one of: a powder composition from which a calcined body with similar processability can be produced without requiring the application of different forming conditions and calcination conditions for each composition; a method for producing the powder composition; a calcined body produced from the powder composition; a method for producing the calcined body; and uses thereof. Furthermore, preferably, the present disclosure can provide at least one of: a method for producing a calcined body capable of reducing variations in productivity; a calcined body produced by the method; and uses thereof.

Description of Embodiments

A powder composition according to the present disclosure is described in the following embodiments.

The present embodiment provides a powder composition comprising: two or more types of zirconia in which a lanthanoid rare-earth element is dissolved; a transition metal element other than zirconium and hafnium; and a remainder composed of zirconia stabilized only by one or more selected from the group consisting of yttrium, calcium and magnesium, wherein a different lanthanoid rare-earth element is dissolved in each zirconia in which the lanthanoid rare-earth element is dissolved, and a transition metal element content is 1500 ppm or less.

The powder composition according to the present embodiment contains two or more types of zirconia in which a lanthanoid rare-earth element is dissolved (hereinafter also referred to as "lanthanoid solid solution zirconia" or "Ln solid solution $ZrO_2$"; zirconia in which erbium is dissolved is also referred to as "erbium solid solution zirconia" or "Er solid solution $ZrO_2$"). A lanthanoid rare-earth element is dissolved in zirconia and is present in zirconia crystals. Thus, Ln solid solution $ZrO_2$ crystals themselves have a color derived from the lanthanoid rare-earth element. Furthermore, in the powder composition according to the present embodiment, in a powder state (that is, a state not subjected to heat treatment that causes thermal shrinkage, such as calcination, after forming zirconia), the lanthanoid rare-earth element is dissolved in zirconia. Thus, unlike a powder composition (mixed powder) containing a lanthanoid rare-earth compound powder and a zirconia powder mixed together, the powder composition according to the present embodiment contains no agglomerate of a lanthanoid rare-earth compound with a particle size of 0.5 $\mu$m or more. Unlike a green body or a calcined body prepared by immersion in a coloring liquid containing a lanthanoid rare-earth element, the powder composition according to the present embodiment is much less likely to have a nonuniform distribution or segregation of a lanthanoid rare-earth element, which causes abnormal growth of zirconia crystal grains during sintering. This reduces the formation of an agglomerate and results in a calcined body with uniform hardness regardless of the lanthanoid rare-earth element content.

The powder composition according to the present embodiment contains two or more types of Ln solid solution $ZrO_2$, and each Ln solid solution $ZrO_2$ contains a different lanthanoid rare-earth element in solid solution. Containing two or more types of Ln solid solution $ZrO_2$ containing a different lanthanoid rare-earth element in solid solution, the powder composition according to the present embodiment can have a composition from which a sintered body with a desired color tone suitable for a dental prosthesis and a calcined body serving as a precursor thereof can be produced.

The powder composition according to the present embodiment may contain two or more types of Ln solid solution $ZrO_2$ and may contain three or more or four or more types of Ln solid solution $ZrO_2$. The powder composition according to the present embodiment may contain any type of Ln solid solution $ZrO_2$ necessary for reproducing the color tone of a natural tooth and may be five or less types of Ln solid solution $ZrO_2$. For example, the powder composition according to the present embodiment is a powder composition containing two types of zirconia containing a different lanthanoid rare-earth element in solid solution, in other words, a powder composition containing two types of zirconia in which a different lanthanoid rare-earth element is dissolved in each zirconia.

The Ln solid solution $ZrO_2$ is preferably zirconia in which one selected from the group consisting of praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm) and ytterbium (Yb) is dissolved, zirconia in which one selected from the group consisting of praseodymium, neodymium, samarium, terbium, dysprosium, holmium, erbium and thulium is dissolved, zirconia in which one selected from the group consisting of praseodymium, neodymium, terbium and erbium is dissolved, zirconia in which one selected from the group consisting of praseodymium, terbium and erbium is dissolved or zirconia in which at least one of terbium and erbium is dissolved. Thus, the powder composition according to the present embodiment is a powder composition containing two or more selected from the group consisting of praseodymium, neodymium, promethium, samarium, europium, gadolinium, terbium, dysprosium, holmium, erbium, thulium and ytterbium, two or more selected from the group consisting of praseodymium, neodymium, samarium, terbium, dysprosium, holmium, erbium and thulium, two or more selected from the group consisting of praseodymium, neodymium, terbium and erbium, two or more selected from the group consisting of praseodymium, terbium and erbium or terbium and erbium. Each Ln solid solution $ZrO_2$ may contain two or more lanthanoid rare-earth elements in solid solution.

Each Ln solid solution $ZrO_2$ may have any lanthanoid rare-earth element content and may have the same lanthanoid rare-earth element content as each lanthanoid solid solution powder described later.

The powder composition according to the present embodiment preferably contains zirconia in which one or more selected from the group consisting of praseodymium, samarium, terbium, dysprosium, holmium and thulium are dissolved, zirconia in which at least one of praseodymium and terbium is dissolved or zirconia in which terbium is dissolved (hereinafter also referred to as "yellowish lanthanoid solid solution zirconia" or "yellow Ln solid solution $ZrO_2$"). When at least one type of Ln solid solution $ZrO_2$ is yellow Ln solid solution $ZrO_2$, that is, when yellow Ln solid solution $ZrO_2$ is contained as Ln solid solution $ZrO_2$, this can facilitate fine adjustment of the color tone of a yellowish tooth in particular.

The powder composition according to the present embodiment preferably contains zirconia in which one or more selected from the group consisting of neodymium and erbium are dissolved, more preferably zirconia in which erbium is dissolved (hereinafter also referred to as "reddish lanthanoid solid solution zirconia" or "red Ln solid solution $ZrO_2$"). When at least one type of Ln solid solution $ZrO_2$ is red Ln solid solution $ZrO_2$, that is, contains red Ln solid solution $ZrO_2$ as Ln solid solution $ZrO_2$, this can facilitate fine adjustment of the color tone of a reddish tooth in particular.

The powder composition according to the present embodiment preferably contains yellow Ln solid solution $ZrO_2$ and red Ln solid solution $ZrO_2$, and Ln solid solution $ZrO_2$ contained in the powder composition according to the present embodiment is preferably yellow Ln solid solution $ZrO_2$ and red Ln solid solution $ZrO_2$.

The powder composition according to the present embodiment may contain Ln solid solution $ZrO_2$ stabilized by one or more selected from the group consisting of yttrium (Y), calcium (Ca) and magnesium (Mg) (hereinafter also referred to as a "stabilizing element"), preferably by yttrium, and at least one type of Ln solid solution $ZrO_2$ is preferably zirconia stabilized by a stabilizing element (hereinafter also referred to as "stabilized Ln solid solution $ZrO_2$"). Lanthanoid rare-earth elements have a function of stabilizing zirconia, and in addition to the stabilization function the coloration increases with the amount of lanthanoid rare-earth element. When Ln solid solution $ZrO_2$ is zirconia stabilized by a stabilizing element and a lanthanoid rare-earth element, the stabilizing element content can be altered to adjust only the stabilizing action without changing the coloration of the Ln solid solution $ZrO_2$. Ln solid solution $ZrO_2$ may be zirconia stabilized only by a lanthanoid rare-earth element. For convenience, the stabilizing element in the present embodiment includes no lanthanoid rare-earth element.

The stabilizing element content of stabilized Ln solid solution $ZrO_2$ may be such an amount that the zirconia is partially stabilized. For example, the yttrium content of Ln solid solution $ZrO_2$ stabilized by yttrium (Y-stabilized Ln solid solution $ZrO_2$) may be 1.5% by mole or more, 2% by mole or more, 3% by mole or more, 3.3% by mole or more, 3.5% by mole or more or 3.6% by mole or more and may be 6.5% by mole or less, 6% by mole or less, 5.5% by mole or less, 5.2% by mole or less or 4.5% by mole or less. The stabilizing element content of stabilized Ln solid solution $ZrO_2$ may be determined as the ratio [% by mole] of the stabilizing element on an oxide basis to the total [mol] of zirconia ($ZrO_2$), the lanthanoid rare-earth element on an oxide basis and the stabilizing element on an oxide basis in the Ln solid solution $ZrO_2$. Stabilizing elements may be based on their respective oxides by substituting $Y_2O_3$ for yttrium, CaO for calcium and MgO for magnesium.

The powder composition according to the present embodiment preferably contains at least yellow stabilized Ln solid solution $ZrO_2$ and red stabilized Ln solid solution $ZrO_2$. Furthermore, stabilized Ln solid solution $ZrO_2$ contained in the powder composition according to the present embodiment is preferably zirconia in which at least one of praseodymium and terbium is dissolved and zirconia in which at least one of neodymium and erbium is dissolved; Tb solid solution $ZrO_2$ and Er solid solution $ZrO_2$; zirconia in which at least one of praseodymium and terbium is dissolved and stabilized by a stabilizing element and by at least one element of praseodymium and terbium and zirconia in which at least one of neodymium and erbium is dissolved; or zirconia in which terbium is dissolved and stabilized by yttrium and terbium, and zirconia in which only erbium is dissolved (that is, Y-stabilized Tb solid solution $ZrO_2$ and Er solid solution $ZrO_2$).

Ln solid solution $ZrO_2$ is preferably a powder, and the powder composition according to the present embodiment preferably contains stabilized Ln solid solution $ZrO_2$ as a powder.

The powder composition according to the present embodiment contains a transition metal element other than zirconium and hafnium (hereinafter also referred to as a "coloring metal element"). For convenience, in the present embodiment, the transition metal element includes no lanthanoid rare-earth element. This facilitates fine adjustment of a color tone that is difficult to obtain using a lanthanoid rare-earth element. The coloring metal element is preferably an element with which the color tone of a grayish tooth can be easily obtained, is a transition metal element other than iron (Fe), is one or more selected from the group consisting of manganese (Mn), cobalt (Co) and titanium (Ti), is two or more selected from the group consisting of manganese, cobalt and titanium, is titanium and at least one of manganese and cobalt, is cobalt and titanium or contains at least titanium.

The coloring metal element contained in the powder composition according to the present embodiment may be in any form and may be in the form of a compound containing the coloring metal element. The coloring metal element may be contained as one or more selected from the group consisting of oxides, hydroxides, oxyhydroxides, chlorides, sulfates and nitrates, one or more selected from the group consisting of oxides, hydroxides and oxyhydroxides or an oxide. Manganese may be contained as one or more selected from the group consisting of MnO, $MnO_2$, $Mn_3O_4$, $Mn(OH)_2$, MnOOH, $MnCl_2$, $MnSO_4$, $Mn(NO_3)_2$ and $Mn(COOH)_2$, one or more selected from the group consisting of MnO, $MnO_2$, $Mn_3O_4$, $Mn(OH)_2$ and MnOOH or one or more selected from the group consisting of MnO, $MnO_2$ and $Mn_3O_4$. Cobalt may be contained as one or more selected from the group consisting of CoO, $CoO_2$, $Co_3O_4$, $Co(OH)_2$, CoOOH, $CoCl_2$, $CoSO_4$, $Co(NO_3)_2$ and CoCOOH, one or more selected from the group consisting of $CoO_2$, $Co_3O_4$, $Co(OH)_2$ and CoOOH, at least one of $CoO_2$ and $Co_3O_4$ or $Co_3O_4$. Titanium may be contained as one or more selected from the group consisting of $TiO_2$, $Ti(OH)_2$, TiOOH, $TiCl_2$, $TiSO_4$, $Ti(NO_3)_2$ and TiCOOH, one or more selected from the group consisting of $TiO_2$, $Ti(OH)_2$ and TiOOH or $TiO_2$. The powder composition according to the present embodiment may contain two or more of these compounds of the coloring metal element.

The powder composition according to the present embodiment contains the remainder composed of zirconia stabilized only by one or more selected from the group consisting of yttrium, calcium and magnesium (hereinafter also referred to as "stabilized zirconia" or "stabilized $ZrO_2$"; zirconia stabilized only by yttrium is also referred to as "yttrium-stabilized zirconia" or "Y-stabilized $ZrO_2$"). Stabilized zirconia is, in particular, zirconia that contains a stabilizing element and in which an element capable of coloring zirconia, such as a lanthanoid rare-earth element, is not dissolved. Stabilized $ZrO_2$ is more preferably zirconia stabilized only by yttrium.

The term "remainder" in the powder composition according to the present embodiment refers to the main component (matrix, parent phase) of the powder composition. Thus, the powder composition according to the present embodiment may be regarded as a powder composition that contains two or more types of zirconia in which a lanthanoid rare-earth element is dissolved and a transition metal element other than zirconium and hafnium and that is composed mainly of zirconia stabilized only by one or more selected from the group consisting of yttrium, calcium and magnesium.

The stabilizing element content of stabilized zirconia may be such an amount that the crystalline phase of the zirconia is partially stabilized. When the stabilizing element is yttrium, the mole ratio of yttrium in terms of $Y_2O_3$ to the total of zirconia ($ZrO_2$) and yttrium in terms of $Y_2O_3$ in yttrium-stabilized zirconia (that is, $\{Y_2O_3 \,[\text{mol}]/ (ZrO_2 + Y_2O_3)\,[\text{mol}]\} \times 100$) is 2.7% by mole or more, 3% by mole or more, 3.3% by mole or more, 3.5% by mole or more or 3.6% by mole or more, and 6.5% by mole or less, 6% by mole or less, 5.5% by mole or less, 5.2% by mole or less or 4.5% by mole or less.

The powder composition according to the present embodiment has a coloring metal element content of 1500 ppm or less (0.15% by mass or less), preferably 1200 ppm or less, 1000 ppm or less, 800 ppm or less, 750 ppm or less or 700 ppm or less. The powder composition according to the present embodiment, which contains a coloring metal element, has a coloring metal element content of more than 0 ppm, preferably 5 ppm or more, 10 ppm or more or 40 ppm or more. In the powder composition according to the present embodiment used as a calcined body, a coloring metal element content above such a range results in nonuniform solid solution or dispersion of the coloring metal element in zirconia, a concentration gradient of the coloring metal element, precipitation of particles in the calcined body or the like, thus resulting in a nonuniform calcined body. A nonuniform calcined body tends to cause a defect, such as chipping or cracking, at the time of processing and has considerable variations in processability from composition to composition. In other words, the coloring metal element content may be such that, in the powder composition according to the present embodiment used as a calcined body, the coloring metal element is uniformly dissolved or dispersed in zirconia and has a color tone that can be applied as a dental prosthetic material.

Among transition metal elements, iron (Fe) tends to form segregation or an agglomerate and tends to result in a calcined body with high hardness. Thus, the powder composition according to the present embodiment preferably contains no iron (that is, the iron content is 0 ppm) but may contain iron without affecting the processability. The iron content is, for example, 0 ppm or more, more than 0 ppm or 1 ppm or more, and 500 ppm or less, 100 ppm or less, 50 ppm or less, 10 ppm or less or 5 ppm or less. In the present embodiment, the iron content is the ratio of the mass of iron in terms of $Fe_2O_3$ to the mass of the powder composition on an oxide basis.

The powder composition according to the present embodiment may contain alumina. The powder composition according to the present embodiment may have an alumina content of 0% by mass or more, more than 0% by mass, 0.005% by mass or more, 0.01% by mass or more or 0.03% by mass or more, and 0.2% by mass or less, 0.1% by mass or less or 0.06% by mass or less. The powder composition according to the present embodiment may contain no alumina (that is, the alumina content may be 0% by mass). The alumina content in the present embodiment is the ratio (% by mass) of the mass of alumina ($Al_2O_3$) to the mass of the powder composition on an oxide basis.

The powder composition according to the present embodiment may contain two or more types of Ln solid solution $ZrO_2$, a coloring metal element, optionally alumina, and the remainder composed of stabilized $ZrO_2$ (that is, the powder composition may contain two or more types of Ln solid solution $ZrO_2$, a coloring metal element, and optionally alumina, and may be composed mainly of stabilized $ZrO_2$), and may contain hafnia ($HfO_2$) as incidental impurities. The hafnia content varies greatly depending on a starting material used in the production of zirconia and a method for producing zirconia and is, for example, 2.0% by mass or less.

In the present embodiment, to calculate the composition, the density or the like using the amount of zirconia ($ZrO_2$), hafnia may be regarded as zirconia.

At a coloring metal element content in such a range, the type and amount of each of Ln solid solution $ZrO_2$, the coloring metal element and stabilized $ZrO_2$ contained in the powder composition according to the present embodiment may be adjusted for the color tone of the intended dental prosthesis.

For example, Ln solid solution $ZrO_2$ is contained such that the powder composition according to the present embodiment has a lanthanoid rare-earth element content of 0.01% by mole or more, 0.02% by mole or more or 0.03% by mole or more, and 0.7% by mole or less, 0.6% by mole or less or 0.5% by mole or less.

The lanthanoid rare-earth element content of the powder composition according to the present embodiment is the ratio of the lanthanoid rare-earth element on an oxide basis [mol] to the total [mol] of zirconia ($ZrO_2$), the lanthanoid rare-earth element on an oxide basis and the stabilizing element on an oxide basis.

For the conversion of lanthanoid rare-earth elements on an oxide basis, praseodymium may be converted in terms of $Pr_6O_{11}$, neodymium may be converted in terms of $Nd_2O_3$, promethium may be converted in terms of $Pm_2O_3$, samarium may be converted in terms of $Sm_2O_3$, europium may be converted in terms of $Eu_2O_3$, gadolinium may be converted in terms of $Gd_2O_3$, terbium may be converted in terms of $Tb_4O_7$, dysprosium may be converted in terms of $Dy_2O_3$, holmium may be converted in terms of $Ho_2O_3$, erbium may be converted in terms of $Er_2Os$, thulium may be converted in terms of $Tm_2O_3$, and ytterbium may be converted in terms of $Yb_2O_3$.

The powder composition according to the present embodiment preferably contains lanthanoid solid solution zirconia such that the ratio [mol/mol] of the total [mol] of neodymium and erbium to the total [mol] of praseodymium, samarium, terbium, dysprosium, holmium and thulium (hereinafter also referred to as the "lanthanoid ratio") as the ratio of the lanthanoid rare-earth element is 3 or more and 60 or less or 5 or more and 48 or less. The visible color tone of a sintered body varies with the transparency/translucency. For example, the lanthanoid ratio may be 10 or more and 30 or less for the color tone of a yellowish tooth (for example, a color tone corresponding to B1 to B4 in the Vita classical shade guide. The same applies hereinafter.). The lanthanoid ratio may be 15 or more and 55 or less or 20 or more and 48 or less for the color tone of a reddish tooth (for example, a color tone corresponding to A1 to A4 in the Vita classical shade guide. The same applies hereinafter.). The lanthanoid ratio may be 3 or more and 35 or less or 5 or more and 30 or less for the color tone of a grayish tooth (for example, a color tone corresponding to C1 to C4 in the Vita classical shade guide. The same applies hereinafter.). The lanthanoid ratio may be 1 or more and 30 or less for the color tone of a dark brownish tooth (for example, a color tone corresponding to D2 to D4 in the Vita classical shade guide. The same applies hereinafter.).

The color tone of the powder composition according to the present embodiment depends on the type of lanthanoid rare-earth element contained therein, and a sintered body therefore has a color tone depending on the combination of lanthanoid rare-earth elements or the like even at the same lanthanoid ratio.

The powder composition according to the present embodiment may have any stabilizing element content depending on the content ratios of the lanthanoid solid solution zirconia and the stabilized zirconia. For example, the powder composition according to the present embodiment has an yttrium content of 2.7% by mole or more, 3% by mole or more, 3.3% by mole or more, 3.5% by mole or more or 3.6% by mole or more, and 6.5% by mole or less, 6% by mole or less, 5.5% by mole or less, 5.2% by mole or less or 4.5% by mole or less.

The Ln solid solution $ZrO_2$ content of the powder composition according to the present embodiment varies with the amount of lanthanoid rare-earth element dissolved in each Ln solid solution $ZrO_2$ and may correspond to the lanthanoid rare-earth element content. The Ln solid solution $ZrO_2$ content of the powder composition according to the present embodiment may be 5% by mass or more and 50% by mass or less, and may be 5% by mass or more and 50% by mass or less or 30% by mass or less for the color tone of a yellowish tooth, 5% by mass or more and 46% by mass or less for the color tone of a reddish tooth, 5% by mass or more and 95% by mass or less or 50% by mass or less for the color tone of a grayish tooth, and 15% by mass or more and 30% by mass or less for the color tone of a dark brownish tooth. Each Ln solid solution $ZrO_2$ content [% by mass] of the powder composition according to the present embodiment is determined from the ratio of the mass of the corresponding Ln solid solution $ZrO_2$ [g] to the mass of the powder composition [g].

Thus, the powder composition according to the present embodiment may have any Ln solid solution $ZrO_2$ content depending on the desired color tone. For example, the Tb solid solution $ZrO_2$ content can be increased to enhance the yellowish color tone, and the Er solid solution $ZrO_2$ content can be increased to enhance the reddish color tone.

For example, the coloring metal element content for the color tone of a reddish tooth may be 50 ppm or more or 70 ppm or more, and 750 ppm or less, 500 ppm or less or 200 ppm or less. The coloring metal element content for the color tone of a yellowish tooth may be 50 ppm or more or 70 ppm or more, and 300 ppm or less or 200 ppm or less. The coloring metal element content for the color tone of a grayish tooth may be 100 ppm or more or 200 ppm or more, and 1100 ppm or less, 950 ppm or less, 800 ppm or less, 500 ppm or less or 300 ppm or less. The coloring metal element content for the color tone of a dark brownish tooth may be 150 ppm or more or 200 ppm or more, and 500 ppm or less, 400 ppm or less or 250 ppm or less.

In the present embodiment, the coloring metal element content is the ratio of the total mass of coloring metal elements on an oxide basis to the mass of the powder composition on an oxide basis. On the oxide basis, manganese, cobalt and titanium may be converted in terms of $MnO_2$, $Co_3O_4$ and $TiO_2$, respectively.

Two or more coloring metal elements, if present, may have any ratio. For example, when cobalt and titanium are contained as coloring metal elements, the ratio [mol/mol] of cobalt (Co) to titanium (Ti) may be 0.01 or more and 1.0 or less or 0.1 or more and 0.3 or less.

When the powder composition according to the present embodiment contains Y-stabilized Pr solid solution $ZrO_2$, Tb solid solution $ZrO_2$, Er solid solution $ZrO_2$, cobalt oxide, manganese oxide, titanium oxide and alumina, with the remainder being Y-stabilized $ZrO_2$ (that is, when the powder composition contains yttrium-stabilized praseodymium solid solution zirconia, terbium solid solution zirconia, erbium solid solution zirconia, cobalt oxide, manganese oxide, titanium oxide and alumina, and is composed mainly of zirconia stabilized only by yttrium), the composition of the powder composition may be regarded as $(Pr_6O_{11} + Tb_4O_7 + Er_2O_3 + Co_3O_4 + TiO_2 + MnO_2 + Al_2O_3 + Y_2O_3 + ZrO_2)$. $Y_2O_3$ in this composition is the sum of $Y_2O_3$ contained in the Y-stabilized Pr solid solution $ZrO_2$ and $Y_2O_3$ contained in the remainder, that is, the Y-stabilized $ZrO_2$. $ZrO_2$ in the composition is the sum of $ZrO_2$ contained in the Y-stabilized Pr solid solution $ZrO_2$, $ZrO_2$ contained in the Tb solid solution $ZrO_2$, $ZrO_2$ contained in the Er solid solution $ZrO_2$ and $ZrO_2$ contained in the remainder, that is, the main component (Y-stabilized $ZrO_2$) of the powder composition. In the powder composition, the lanthanoid rare-earth element content [% by mole] is determined using $\{ (Pr_6O_{11} + Tb_4O_7 + Er_2O_3)$ [mol] / $(Pr_6O_{11} + Tb_4O_7 + Er_2O_3 + Y_2O_3 + ZrO_2)$ [mol] $\} \times 100$, the stabilizing element content [% by mole] is determined using $\{Y_2O_3$ [mol] / $(Pr_6O_{11} + Tb_4O_7 + Er_2O_3 + Y_2O_3 + ZrO_2)$ [mol] $\} \times 100$, the coloring metal element content [ppm] is determined using $\{ (TiO_2 + Co_3O_4 + MnO_2)$ [g] / $(Pr_6O_{11} + Tb_4O_7 + Er_2O_3 + Co_3O_4 + TiO_2 + MnO_2 + Al_2O_3 + Y_2O_3 + ZrO_2)$ [g] $\} \times 10^6$, and the alumina content [% by mass] is determined using $\{Al_2O_3$ [g] / $(Pr_6O_{11} + Tb_4O_7 + Er_2O_3 + Co_3O_4 + TiO_2 + MnO_2 + Al_2O_3 + Y_2O_3 + ZrO_2)$ [g] $\} \times 100$. The lanthanoid ratio [mol/mol] is determined using $\{ (Er_2O_3)$ [mol] / $(Pr_6O_{11} + Tb_4O_7)$ [mol] $\}$. The same applies to the case where the powder composition contains a binder. Each Ln solid solution $ZrO_7$ content [% by mass] of the powder composition is determined from the ratio of the mass of the corresponding Ln solid solution $ZrO_7$ [g] to the mass of the powder composition [g].

The powder composition according to the present embodiment may contain a transition metal element, a lanthanoid rare-earth element and a stabilizing element that are not dissolved in zirconia, provided that the advantages of the powder composition are not impaired. However, these elements are preferably not contained, and, more preferably, at least a powder X-ray diffraction (hereinafter also referred to as "XRD") pattern of the powder composition does not have an XRD peak corresponding to a transition metal compound, a lanthanoid rare-earth compound or a compound of a stabilizing element.

The XRD measurement conditions in the present embodiment include the following conditions.

Radiation source: CuKα radiation (λ = 0.15418 nm)
Measurement mode: continuous scanning
Scanning speed: 4 degrees/min
Measurement range: 2θ = 26 to 33 degrees
Accelerating voltage and electric current: 40 mA and 40 kV
Divergence height limiting slit: 10 mm
Divergence/incident slit: 1 degree
Light-receiving slit: open
Detector: semiconductor detector (D/teX Ultra)
Filter: Ni filter
Goniometer radius: 185 mm

XRD measurement can be performed with a typical X-ray diffractometer (for example, Ultima IV, manufactured by Rigaku Corporation).

The powder composition according to the present embodiment preferably has a crystalline phase of zirconia and may only has a crystalline phase of zirconia. Furthermore, the crystalline phase of the powder according to the present embodiment preferably contains at least one of tetragonal zirconia and cubic zirconia, more preferably monoclinic zirconia and at least one of tetragonal zirconia and cubic zirconia. The monoclinic zirconia content of the crystalline phase of the powder composition according to the present embodiment (hereinafter also referred to as the "monoclinic ratio") may be 0% or more, more than 0%, 3% or more or 5% or more, and 40% or less, 35% or less, 25% or less, 15% or less, 10% or less or 8% or less. Crystalline phases other than monoclinic zirconia in the crystalline phase of the powder composition according to the present embodiment may be regarded as tetragonal zirconia and cubic zirconia.

Crystalline phases can be examined by XRD measurement under the conditions described above. An XRD peak corresponding to each crystal plane of zirconia may be an XRD peak with a peak top at the 2θ described below.

XRD peak corresponding to (111) plane of monoclinic zirconia: 2θ = 31 ± 0.5 degrees
XRD peak corresponding to (11-1) plane of monoclinic zirconia: 2θ = 28 ± 0.5 degrees
XRD peak corresponding to (111) plane of tetragonal zirconia: 2θ = 30 ± 0.5 degrees
XRD peak corresponding to (111) plane of cubic zirconia: 2θ = 30 ± 0.5 degrees
The monoclinic ratio is a value obtained from an XRD pattern of a powder composition using the following formula.

$$f_M = [1 - \{[It(111) + Ic(111)]/[Im(111) + Im(11-1) + It(111) + Ic(111)]\}] \times 100$$

In this formula, $f_M$ denotes the monoclinic ratio [%], It(111) denotes the integrated intensity of an XRD peak corresponding to the tetragonal zirconia (111) plane, Ic(111) denotes the integrated intensity of an XRD peak corresponding to the cubic zirconia (111) plane, Im(111) denotes the integrated intensity of an XRD peak corresponding to the monoclinic zirconia (111) plane, and Im(11-1) denotes the integrated intensity of an XRD peak corresponding to the monoclinic zirconia (11-1) plane. The XRD peak corresponding to the tetragonal zirconia (111) plane and the XRD peak corresponding to the cubic zirconia (111) plane are measured as one overlapping peak (hereinafter also referred to as a "main XRD peak"). Thus, It(111) + Ic(111) in the formula corresponds to the integrated intensity of one XRD peak with a peak top at 2θ = 30 ± 0.5 degrees.

The integrated intensity of each crystal plane can be determined by profile fitting of an XRD pattern after smoothing treatment and background subtraction treatment using a split pseudo-Voigt function. XRD pattern analyses, such as the smoothing treatment, the background treatment, and the calculation of the integrated intensity, may be performed using an analysis program attached to an X-ray diffractometer (for example, integrated X-ray powder diffraction software PDXL Ver. 2.2, manufactured by Rigaku Corporation).

The powder composition according to the present embodiment may have a crystallite size of 300 nm or more, 350 nm or more or 370 nm or more, and 450 nm or less or 400 nm or less.

In the present embodiment, the crystallite size of a powder composition may be the crystallite size of zirconia determined from a main XRD peak. The crystallite size of a powder composition is a value calculated using the following

formula:

$$D = \kappa\lambda/\beta\cos\theta$$

wherein D denotes the crystallite size (angstroms), $\kappa$ denotes the Scherrer constant ($\kappa = 1$), $\lambda$ denotes the wavelength of a measurement X-ray ($\lambda$, = 0.15418 nm when CuK$\alpha$ radiation is used as a radiation source), $\beta$ denotes the half-width (degrees) of a main XRD peak, and $\theta$ denotes the Bragg angle of the main XRD peak. The half-width of a main XRD peak is the value of the half-width of the main XRD peak determined by profile fitting of an XRD pattern after the smoothing treatment and the background subtraction treatment using the split pseudo-Voigt function. XRD pattern analyses, such as the smoothing treatment, the background treatment and the profile fitting, may be performed using an analysis program attached to an X-ray diffractometer (for example, integrated X-ray powder diffraction software PDXL Ver. 2.2, manufactured by Rigaku Corporation).

The powder composition according to the present embodiment may have a BET specific surface area of 5 m$^2$/g or more and 15 m$^2$/g or less, preferably 7 m$^2$/g or more, 9 m$^2$/g or more, 9.5 m$^2$/g or more or 10 m$^2$/g or more, and preferably 13 m$^2$/g or less, 12 m$^2$/g or less or 11 m$^2$/g or less.

In the present embodiment, the BET specific surface area is a BET specific surface area measured in accordance with JIS Z 8830 and may be measured by a BET one-point method using a carrier gas method using nitrogen as an adsorption gas. The specific measurement conditions of the BET specific surface area may be as follows:

Adsorption medium: N$_2$
Adsorption temperature: -196°C
Pretreatment conditions: in air at 250°C for 30 minutes
The BET specific surface area can be measured with a general apparatus (for example, FlowSorb III 2305, manufactured by Shimadzu Corporation).

The powder composition according to the present embodiment preferably has an average particle size of 0.2 $\mu$m or more, 0.3 $\mu$m or more or 0.4 $\mu$m or more, and 0.7 $\mu$m or less, 0.6 um or less, 0.5 $\mu$m or less.

In the present embodiment, the average particle size is $D_{50}$ (median size) in a volumetric particle size distribution of a powder composition measured by a wet method and can be measured with a general apparatus. The measurement sample may be a slurry prepared by dispersing, in pure water, a powder composition from which slow aggregation has been removed by ultrasonication or another dispersion treatment. A preferred method for measuring the average particle size and the conditions therefor may be as follows:

Measuring apparatus: MT3300EXII
Calculation mode: HRA
Particle refractive index: 2.17
Solvent refractive index: 1.333
Particle shape: nonspherical
Measurement sample: slurry of powder composition (solvent: pure water)

The powder composition according to the present embodiment may contain a binding agent (binder), if necessary. The formability can be adjusted with a binding agent. The binding agent may be one or more selected from the group consisting of poly(vinyl alcohol), poly(vinyl butyrate), waxes and acrylic resins, preferably one or more of poly(vinyl alcohol) and acrylic resins, more preferably an acrylic resin. In the present embodiment, acrylic resins are polymers containing at least one of acrylate and methacrylate. A specific acrylic resin may be one or more selected from the group consisting of poly(acrylic acid), poly(methacrylic acid), acrylic acid copolymers and methacrylic acid copolymers or a derivative or derivatives thereof.

The powder composition according to the present embodiment preferably contains granular particles and is preferably a granular powder. In the present embodiment, the "granular particles" are particles in a state where secondary particles of a powder are slowly aggregated by physical force, and preferably have a particle size of 25 $\mu$m or more, more preferably 25 $\mu$m or more and 180 $\mu$m or less, still more preferably 25 $\mu$m or more and 125 $\mu$m or less. The granular particles may contain a binding agent, such as an acrylic resin, if necessary. The "granular powder" is a powder composed mainly of granular particles, preferably a powder composed of granular particles. The powder composition according to the present embodiment containing granular particles or being a granular powder has improved operability (handleability).

The powder composition according to the present embodiment preferably contains granular particles composed of a transition metal element (coloring metal element) other than zirconium and hafnium, and zirconia stabilized only by one or more selected from the group consisting of yttrium, calcium and magnesium (stabilized zirconia). In the granular particles composed of the coloring metal element and the stabilized zirconia, the diffusion of the coloring metal element and the solid solution of the coloring metal element into the stabilized zirconia are promoted during heat treatment, and the precipitation of a compound of the coloring metal element is more easily suppressed.

The powder composition according to the present embodiment is preferably a granular powder containing two or more types of granular particles composed of Ln solid solution $ZrO_2$, and granular particles containing a transition metal element other than zirconium and hafnium with the remainder being composed of stabilized $ZrO_2$ (that is, two or more types of granular particles composed of zirconia in which a lanthanoid rare-earth element is dissolved, and granular particles that contain a transition metal element other than zirconium and hafnium and that are composed of powder particles composed mainly of zirconia stabilized only by one or more selected from the group consisting of yttrium, calcium and magnesium), more preferably a granular powder composed of two or more types of granular particles composed of Ln solid solution $ZrO_2$, and granular particles containing a transition metal element other than zirconium and hafnium with the remainder being composed of stabilized $ZrO_2$ (that is, two or more types of granular particles composed of zirconia in which a lanthanoid rare-earth element is dissolved, and granular particles that contain a transition metal element other than zirconium and hafnium and that are composed of powder particles composed mainly of zirconia stabilized only by one or more selected from the group consisting of yttrium, calcium and magnesium).

In the powder composition according to the present embodiment containing alumina, alumina may be uniformly dispersed in the powder composition. Alumina may be contained in any form, for example, in at least one type of granular particles or in all types of granular particles, or granular particles may be composed only of alumina.

The powder composition according to the present embodiment may have an average granule size of 20 $\mu$m or more, 30 $\mu$m or more or 40 $\mu$m or more, and 100 $\mu$m or less, 80 $\mu$m or less, 60 $\mu$m or less or 50 $\mu$m or less. The average granule size can be determined as a granule size at 50% by mass in a cumulative granule size curve obtained by plotting the granule size and the mass ratio, which are obtained by a mechanical sieving method using a typical Ro-Tap sieve shaker (for example, sieve shaker S-1, manufactured by Teraoka). In the mechanical sieving method, sieves with apertures of 125 pm, 106 pm, 90 pm, 75 pm, 63 pm, 45 pm, 38 $\mu$m and 25 $\mu$m may be stacked in this order in accordance with JIS Z 8801. The conditions for the mechanical sieving method may be as follows:

Shaking frequency: 300 rpm
Shaking width: 25 mm
Standard number of blows of hammer: 150 rpm
Shaking time: 30 minutes

Before the measurement, a granule sample is preferably lightly loosened to the extent that the granule sample can pass through a sieve with an aperture of 125 $\mu$m or less.

The cumulative granule size curve is obtained by plotting the granule size and the mass ratio. The granule size of a powder composition remaining on each sieve after shaking is regarded as the granule size equivalent to the opening size of the smallest aperture through which granular particles have passed (for example, the granule size of granular particles passing through a sieve with an aperture of 90 $\mu$m and remaining on a sieve with an aperture of 75 $\mu$m is regarded as 90 pm).

A calcined body suitable as a precursor of a dental prosthesis can be produced from the powder composition according to the present embodiment by an industrial production method, such as uniaxial pressing, CIP treatment or a forming method equivalent thereto, a calcination method in air at 950°C or more and less than 1200°C or a calcination method in air at 950°C or more and 1100°C or less for 0.5 hours or more and 10 hours or less.

Next, a method for producing the powder composition according to the present embodiment is described.

The powder composition according to the present embodiment can be produced by any method for producing a powder composition satisfying the constitution described above. A preferred method for producing a powder composition may include the step of mixing two or more types of powders of zirconia in which a lanthanoid rare-earth element is dissolved, a powder of a transition metal compound other than zirconium and hafnium, and a powder of zirconia stabilized only by one or more selected from the group consisting of yttrium, calcium and magnesium, such that the transition metal element content is 1500 ppm or less (hereinafter also referred to as a "mixing step"), wherein a different lanthanoid rare-earth element is dissolved in each of the powders of the zirconia in which the lanthanoid rare-earth element is dissolved.

Powders to be subjected to the mixing step are two or more types of powders of zirconia in which a lanthanoid rare-earth element is dissolved (hereinafter also referred to as "lanthanoid solid solution powders" or "Ln solid solution powders"; when the lanthanoid is erbium, the powders are also referred to as "erbium solid solution powders" or "Er solid solution powders"), a powder of a transition metal compound other than zirconium and hafnium (hereinafter also referred to as a "coloring metal powder") and a powder of zirconia stabilized only by one or more selected from the group consisting of yttrium, calcium and magnesium (hereinafter also referred to as a "stabilized powder").

All of the Ln solid solution powders, the coloring metal powder and the stabilized powder (hereinafter collectively referred to as "raw material powders") preferably have physical properties similar to the physical properties of the powder composition according to the present embodiment. More preferably, the Ln solid solution powders and the stabilized powder, which account for a large proportion of the powder composition according to the present embodiment, have similar physical properties. On the other hand, the coloring metal powder preferably has physical properties similar to the physical properties of these. However, accounting for a small proportion of the powder composition according to the present embodiment and therefore having a small influence, the coloring metal powder may have physical properties different from the physical properties of the other raw material powders.

Examples of preferred physical properties of the Ln solid solution powders and the stabilized powder include the following physical properties.

BET specific surface area: 5 $m^2/g$ or more, 9 $m^2/g$ or more, 9.5 $m^2/g$ or more or 10 $m^2/g$ or more, and 15 $m^2/g$ or less, 13 $m^2/g$ or less, 12 $m^2/g$ or less or 11 $m^2/g$ or less, and

average particle size: 0.2 $\mu m$ or more, 0.3 $\mu m$ or more or 0.4 $\mu m$ or more, and 0.7 $\mu m$ or less, 0.6 $\mu m$ or less or 0.5 $\mu m$ or less.

The difference between the maximum value and the minimum value of the BET specific surface areas of the Ln solid solution powders and the stabilized powder to be subjected to the mixing step (hereinafter also referred to as the "BET difference") may be 0 $m^2/g$ or more, more than 0 $m^2/g$ or 0.1 $m^2/g$ or more, and 3.5 $m^2/g$ or less, 2.0 $m^2/g$ or less, 0.7 $m^2/g$ or less, 0.5 $m^2/g$ or less or 0.3 $m^2/g$ or less. The difference between the maximum value and the minimum value of the average particle size of the raw material powders (hereinafter also referred to as the "particle size difference") is 0 $\mu m$ or more, more than 0 $\mu m$ or 0.05 $\mu m$ or more, and 0.5 $\mu m$ or less, 0.3 $\mu m$ or less or 0.1 $\mu m$ or less. A smaller BET difference and a smaller particle size difference tend to result in the powder composition according to the present embodiment with more uniform physical properties.

Each Ln solid solution powder may be a powder of zirconia in which a lanthanoid rare-earth element is dissolved with a desired coloration and may be a powder of zirconia in which one or more selected from the group consisting of praseodymium, neodymium, promethium, samarium, europium, gadolinium, terbium, dysprosium, holmium, erbium, thulium and ytterbium are dissolved, preferably a powder of zirconia in which one or more selected from the group consisting of praseodymium, neodymium, samarium, terbium, dysprosium, holmium, erbium and thulium are dissolved, a powder of zirconia in which one or more selected from the group consisting of praseodymium, neodymium, terbium and erbium are dissolved or a powder of zirconia in which at least one of terbium and erbium is dissolved (a powder of zirconia in which erbium is dissolved is hereinafter also referred to as an "erbium solid solution powder" or an "Er solid solution powder").

Two or more, three or more or four or more types and five or less types of Ln solid solution powders in which a different lanthanoid rare-earth element is dissolved may be used.

At least one of the Ln solid solution powders may be stabilized by one or more selected from the group consisting of yttrium, calcium and magnesium (stabilizing element(s)), preferably yttrium.

The Ln solid solution powders contained in the powder composition according to the present embodiment preferably contains a powder of zirconia in which at least one of praseodymium and terbium is dissolved and that is stabilized by a stabilizing element and by at least one of praseodymium and terbium and a powder of zirconia in which at least one of neodymium and erbium is dissolved and more preferably contains a powder of zirconia in which terbium is dissolved and that is stabilized by yttrium and terbium (Y-stabilized Tb solid solution $ZrO_2$) and a powder of zirconia in which only erbium is dissolved (Er solid solution $ZrO_2$).

Each Ln solid solution powder has any lanthanoid rare-earth element content, which can also be determined as the ratio of the lanthanoid rare-earth element on an oxide basis [mol] to the total [mol] of zirconia, the lanthanoid rare-earth element on an oxide basis and the stabilizing element in the Ln solid solution powder. For example, the terbium content of a Tb solid solution powder ($\{Tb_4O_7$ [mol]/($Tb_4O_7 + ZrO_2$) [mol]$\} \times 100$) [% by mole] may be 0.0005% by mole or more, 0.005% by mole or more or 0.03% by mole or more, and 0.10% by mole or less, 0.06% by mole or less or 0.05% by mole or less. The terbium content of an Y-stabilized Tb solid solution powder ($\{Tb_4O_7$ [mol] /($Tb_4O_7 + ZrO_2 + Y_2O_3$) [mol] $\} \times 100$) [% by mole] may be 0.0005% by mole or more, 0.002% by mole or more, 0.005% by mole or more or 0.03% by mole or more, and 0.10% by mole or less, 0.06% by mole or less or 0.05% by mole or less.

The erbium content of an Er solid solution powder ($\{Er_2O_3$ [mol]/($Er_2O_3 + ZrO_2$) [mol]$\} \times 100$) [% by mole] may be 1.5% by mole or more, 2.0% by mole or more or 3.3% by mole or more, and 6% by mole or less, 5.0% by mole or less or 4.5% by mole or less.

The praseodymium content of a Pr solid solution powder ($\{Pr_6O_{11}$ [mol] / ($Pr_6O_{11} + ZrO_2$) [mol] $\} \times 100$) [% by mole] may be 0.05% by mole or more, 0.07% by mole or more or 0.1% by mole or more, and 1.0% by mole or less, 0.6% by mole or less, 0.5% by mole or less or 0.4% by mole or less. The praseodymium content of an Y-stabilized Pr solid solution powder ($\{Pr_6O_{11}$ [mol]/($Pr_6O_{11} + ZrO_2 + Y_2O_3$) [mol] $\} \times 100$) [% by mole] may be 0.05% by mole or more, 0.07% by mole or more or 0.1% by mole or more, and 1.2% by mole or less, 1.0% by mole or less or 0.8% by mole or less.

The neodymium content of a Nd solid solution powder ($\{Nd_2O_3 \text{ [mol]}/(Nd_2O_3 + ZrO_2) \text{ [mol]}\} \times 100$) [% by mole] may be 0.05% by mole or more, 0.07% by mole or more or 0.1% by mole or more, and 2.0% by mole or less, 1.8% by mole or less or 1.6% by mole or less. The neodymium content of an Y-stabilized Nd solid solution powder ($\{Nd_2O_3 \text{ [mol]}/(Nd_2O_3 + ZrO_2 + Y_2O_3) \text{ [mol]}\} \times 100$) [% by mole] may be 0.05% by mole or more, 0.07% by mole or more or 0.1% by mole or more, and 2.0% by mole or less, 1.8% by mole or less or 1.6% by mole or less.

The Ln solid solution powders may contain any amount of stabilizing element. When the stabilizing element is yttrium, the yttrium content may be 1.5% by mole or more, 2% by mole or more, 3% by mole or more, 3.3% by mole or more, 3.5% by mole or more or 7% by mole or more, and 6.5% by mole or less, 6% by mole or less, 5.5% by mole or less or 5.2% by mole or less, as a ratio of yttrium in terms of $Y_2O_3$ [mol] to the total [mol] of zirconia ($ZrO_2$), the lanthanoid rare-earth element on an oxide basis and yttrium in terms of $Y_2O_3$ in the Ln solid solution powders.

The stabilized powder is a powder of zirconia stabilized only by one or more selected from the group consisting of yttrium, calcium and magnesium. The stabilized powder is preferably a powder of zirconia that contains an element with the function of stabilizing zirconia and in which no lanthanoid rare-earth element with the function of coloring zirconia is dissolved. The stabilized powder is more preferably a powder of zirconia stabilized only by yttrium.

The stabilizing element content of the stabilized powder may be such an amount that zirconia is partially stabilized. When the stabilizing element is yttrium, the mole ratio of yttrium in terms of $Y_2O_3$ to the total of zirconia ($ZrO_2$) and yttrium in terms of $Y_2O_3$ in the stabilized powder (that is, $\{Y_2O_3 \text{ [mol]} / (ZrO_2 + Y_2O_3) \text{ [mol]}\} \times 100$ [% by mole]) is 2.7% by mole or more, 3% by mole or more, 3.3% by mole or more, 3.5% by mole or more or 7% by mole or more, and 6.5% by mole or less, 6% by mole or less, 5.5% by mole or less, 5.2% by mole or less or 4.5% by mole or less.

The Ln solid solution powders and the stabilized powder may be produced by any method, for example, by a production method including the step of heat-treating a mixture of a zirconia sol and at least one of a stabilizing element source and a lanthanoid source at 950°C or more and 1250°C or less and then grinding the mixture.

The stabilizing element source is a compound containing a stabilizing element and may be one or more selected from the group consisting of oxides, hydroxides, oxyhydroxides, halides, sulfates and nitrates of the stabilizing element, one or more selected from the group consisting of oxides, hydroxides and chlorides of the stabilizing element, at least one of oxides and chlorides of the stabilizing element, or a chloride.

A specific stabilizing element source is, for example, one or more selected from the group consisting of yttrium oxide, yttrium hydroxide, yttrium chloride, yttrium bromide, yttrium boride, yttrium iodide, yttrium sulfate and yttrium nitrate; one or more selected from the group consisting of calcium oxide, calcium hydroxide, calcium chloride, calcium bromide, calcium boride, calcium iodide, calcium sulfate and calcium nitrate; or one or more selected from the group consisting of magnesium oxide, magnesium hydroxide, magnesium chloride, magnesium bromide, magnesium boride, magnesium iodide, magnesium sulfate and magnesium nitrate. A preferred stabilizing element source may be one or more selected from the group consisting of yttrium oxide, yttrium hydroxide, yttrium oxyhydroxide, yttrium chloride, yttrium bromide, yttrium boride, yttrium iodide, yttrium sulfate and yttrium nitrate; one or more selected from the group consisting of yttrium oxide, yttrium hydroxide, yttrium chloride and yttrium bromide; one or more selected from the group consisting of yttrium oxide, yttrium hydroxide and yttrium chloride; at least one of yttrium oxide and yttrium chloride; or yttrium chloride.

The lanthanoid source is a compound containing a lanthanoid rare-earth element and may be one or more selected from the group consisting of oxides, hydroxides, oxyhydroxides, halides, sulfates and nitrates of a lanthanoid rare-earth element, one or more selected from the group consisting of oxides, hydroxides and chlorides of a lanthanoid rare-earth element, at least one of oxides and chlorides of a lanthanoid rare-earth element or a chloride.

The lanthanoid source is, for example, one or more selected from the group consisting of oxides, hydroxides, oxyhydroxides, halides, sulfates and nitrates containing one or more selected from the group consisting of praseodymium, neodymium, promethium, samarium, europium, gadolinium, terbium, dysprosium, holmium, erbium, thulium and ytterbium. A lanthanoid source containing erbium (a lanthanoid source containing erbium is hereinafter also referred to as an "erbium source") may be one or more selected from the group consisting of erbium oxide, erbium hydroxide, erbium oxyhydroxide, erbium chloride, erbium bromide, erbium boride, erbium iodide, erbium sulfate and erbium nitrate, one or more selected from the group consisting of erbium oxide, erbium hydroxide and erbium chloride, erbium oxide or erbium chloride, or erbium chloride. A terbium source may be one or more selected from the group consisting of terbium oxide, terbium hydroxide, terbium chloride, terbium bromide, terbium boride, terbium iodide, terbium sulfate and terbium nitrate, one or more selected from the group consisting of terbium oxide, terbium hydroxide and terbium chloride, terbium oxide or terbium chloride, or terbium chloride. The same applies to a lanthanoid source containing another lanthanoid rare-earth element, and at least one of oxides and chlorides or a chloride is preferred.

The zirconia sol is preferably a zirconia sol produced by any one of a hydrothermal synthesis method and a hydrolysis method, more preferably a zirconia sol produced by a hydrolysis method. The zirconia sol may be in a hydrated state.

In the production of the Ln solid solution powders, the lanthanoid source and, if necessary, the stabilizing element source may be mixed with the zirconia sol. On the other hand, in the production of the stabilized powder, the stabilizing element source and the zirconia sol may be mixed. This results in a mixture.

The mixing method may be any known method of homogeneously mixing the stabilizing element source or the lanthanoid source with zirconia. The mixing method may be at least one of wet mixing and dry mixing or may be wet mixing. In wet mixing, the solvent may be at least one of water and alcohols, preferably a solvent containing at least ethanol, or ethanol.

The resulting mixture is heat treated at 950°C or more or 1000°C or more, and 1250°C or less or 1200°C or less. The BET specific surface area tends to decrease as the heat-treatment temperature increases. Thus, to achieve a desired BET specific surface area, the heat-treatment temperature, the heat-treatment time and the temperature increase/decrease rate may be appropriately set in the temperature range described above depending on the treatment amount, a heat treatment method used and a heat treatment furnace used.

The Ln solid solution powders and the stabilized powder are each produced by grinding the mixture after the heat treatment. The grinding method may be any known method that can produce a powder with a desired particle size. The grinding method may be at least one of wet grinding and dry grinding or may be wet grinding. In wet grinding, the solvent may be at least one of water and alcohols, preferably an alcohol. The grinding time depends on the grinding method, the amount of powder to be ground and the like. The particle size of a powder composition before reaching its equilibrium tends to decrease as the amount of powder to be ground and the grinding time increase, and the grinding conditions may be appropriately adjusted for the target particle size.

The heat treatment temperature and the grinding time can be appropriately altered to adjust the BET specific surface area and the particle size of the powder.

The coloring metal powder is a powder of a transition metal compound other than zirconium and hafnium (a powder of a compound of a coloring metal element) and may be contained as it is in the powder composition according to the present embodiment. Preferably, the coloring metal powder is a powder of at least one of oxides, hydroxides, oxyhydroxides, chlorides, sulfates and nitrates of a coloring metal element or a powder of at least one of oxides, hydroxides and oxyhydroxides of a coloring metal element. For example, the coloring metal powder containing manganese as a coloring metal element may be one or more powders selected from the group consisting of $MnO$, $MnO_2$, $Mn_3O_4$, $Mn(OH)_2$, $MnOOH$, $MnCl_2$, $MnSO_4$, $Mn(NO_3)_2$ and $Mn(COOH)_2$, one or more powders selected from the group consisting of $MnO$, $MnO_2$, $Mn_3O_4$, $Mn(OH)_2$ and $MnOOH$ or one or more powders selected from the group consisting of $MnO$, $MnO_2$ and $Mn_3O_4$. The coloring metal powder containing cobalt as a coloring metal element may be one or more powders selected from the group consisting of $CoO$, $CoO_2$, $Co_3O_4$, $Co(OH)_2$, $CoOOH$, $CoCl_2$, $CoSO_4$, $Co(NO_3)_2$ and $CoCOOH$, one or more powders selected from the group consisting of $CoO_2$, $Co_3O_4$, $Co(OH)_2$ and $CoOOH$ or at least one powder of $CoO_2$ and $Co_3O_4$. The coloring metal powder containing titanium as a coloring metal element may be one or more powders selected from the group consisting of $TiO_2$, $Ti(OH)_2$, $TiOOH$, $TiCl_2$, $TiSO_4$, $Ti(NO_3)_2$ and $TiCOOH$, one or more powders selected from the group consisting of $TiO_2$, $Ti(OH)_2$ and $TiOOH$ or a powder of $TiO_2$.

The coloring metal powder may be in such an amount that the coloring metal element content of the powder composition produced by the mixing step is 1500 ppm or less and may be in any amount such that the coloring metal element content is 1500 ppm or less, for example, more than 0 ppm, 5 ppm or more, 10 ppm or more or 40 ppm or more, and 1500 ppm or less, 1200 ppm or less, 1000 ppm or less, 800 ppm or less, 750 ppm or less or 700 ppm or less.

In addition to the raw material powders, if necessary, an alumina source may be subjected to the mixing step. The alumina source may be at least one of alumina and a precursor thereof, for example, at least one of alumina and aluminum hydroxide or alumina. The alumina source content may be the same as the alumina content of a desired powder composition. The alumina source may be contained in at least one of the Ln solid solution powders and the stabilized powder.

In the mixing step, no iron compound powder is preferably contained. However, each raw material powder may contain iron as an incidental impurity.

In the mixing step, the raw material powders and, if necessary, an alumina source may be mixed such that the transition metal element content is 1500 ppm or less. A desired powder composition can be produced by appropriately selecting the type and amount of each raw material powder to be mixed. For example, to form a sintered body with a color tone with low saturation (for example, a color tone corresponding to A1 to A2, B1 to B2, C1 to C2 or D2 in the Vita classical shade guide), the stabilized powder may be mixed at a high ratio. Similarly, to form a sintered body with the color tone of a reddish tooth, a powder of zirconia in which at least one of neodymium and erbium is dissolved, preferably a powder of zirconia in which erbium is dissolved, may be used as a lanthanoid solid solution powder and may be mixed at a high ratio. To form a sintered body with the color tone of a yellowish tooth, a powder in which praseodymium and terbium are dissolved may be used as a lanthanoid solid solution powder and may be mixed at a high ratio. To form a sintered body with the color tone of a grayish tooth, the coloring metal powder may be mixed at a high ratio.

Any mixing method may be used, and at least one of dry mixing and wet mixing may be used. Dry mixing may be used when the mixing efficiency is regarded as important, and wet mixing may be used when high homogeneity is regarded as important.

The method for producing the powder composition according to the present embodiment may include at least one of the step of granulating the raw material powders before the mixing step and the step of granulating the powder composition after the mixing step, preferably the step of granulating each raw material powder before the mixing step (hereinafter the former is also referred to as a "pre-granulation step" and the latter is also referred to as a "post-granulation step", which are collectively referred to as a "granulation step").

In the pre-granulation step, instead of or in addition to each raw material powder, a granular powder containing each raw material powder may be subjected to the mixing step. In this case, a granular powder to be subjected to the mixing step is, for example, one or more selected from the group consisting of a granular powder of zirconia in which a lanthanoid rare-earth element is dissolved (Ln solid solution $ZrO_2$), a granular powder of zirconia in which a lanthanoid rare-earth element is dissolved and that is stabilized by a stabilizing element and a lanthanoid rare-earth element (stabilized Ln solid solution $ZrO_2$), a granular powder of a transition metal compound other than zirconium and hafnium, a granular powder of zirconia stabilized only by one or more selected from the group consisting of yttrium, calcium and magnesium (stabilized $ZrO_2$), a granular powder that contains a transition metal compound other than zirconium and hafnium with the remainder being composed of zirconia stabilized only by one or more selected from the group consisting of yttrium, calcium and magnesium (that is, a granular powder that contains a transition metal compound other than zirconium and hafnium and is composed of a powder composed mainly of zirconia stabilized only by one or more selected from the group consisting of yttrium, calcium and magnesium) and a granular powder of Ln solid solution $ZrO_2$ containing a transition metal compound other than zirconium and hafnium. At least a granular powder containing a compound of a coloring metal element with the remainder being granular particles of zirconia stabilized only by a stabilizing element (that is, granular particles that contain a compound of a coloring metal element and are composed of a powder composed mainly of zirconia stabilized only by a stabilizing element) is preferably subjected to the mixing step.

The average granule size of each granular powder produced by the pre-granulation step is preferably the same as the average granule size of the powder composition according to the present embodiment. The granular powders preferably have almost the same average granule size and may have a difference between the maximum average granule size and the minimum average granule size of 0 $\mu$m or more, more than 0 $\mu$m or 1 $\mu$m or more, and 10 $\mu$m or less, 8 $\mu$m or less or 5 $\mu$m or less.

In the post-granulation step, if present, a powder composition produced by the mixing step may be granulated.

The granulation method in the granulation step may be any method in which secondary particles of the powder (the raw material powders or the powder composition) are slowly aggregated and form granular particles. The granulation method may be one or more selected from the group consisting of a spray drying method, an agitation granulation method and an extrusion granulation method or a spray drying method. In the spray drying method, a slurry of a powder to be granulated dispersed in a solvent is spray-dried as droplets to produce a granular powder. The solvent may be at least one of water and alcohols. If necessary, the slurry may be mixed with a binding agent, such as an acrylic resin, before spray drying and granulation. For example, granular particles that contain a transition metal element other than zirconium and hafnium with the remainder being composed of zirconia stabilized only by one or more selected from the group consisting of yttrium, calcium and magnesium (that is, contain a transition metal element other than zirconium and hafnium and are composed mainly of zirconia stabilized only by one or more selected from the group consisting of yttrium, calcium and magnesium) may be mixed with a stabilized powder, a coloring metal powder and a solvent to prepare a slurry, which is then spray-dried. In the spray drying method, the particle size of the resulting granular particles tends to increase with the concentration of powder in the slurry. Thus, the granule size in spray drying may be appropriately adjusted by the concentration of powder in the slurry.

Next, a method for producing a calcined body using the powder composition according to the present embodiment is described below.

A calcined body can be produced from the powder composition according to the present embodiment by a method for producing a calcined body including the step of calcining a green body composed of the powder composition according to the present embodiment.

A green body to be subjected to the step of calcining a green body composed of the powder composition according to the present embodiment (hereinafter also referred to as the "calcination step") is a green compact or a green compact in a state where the powder composition according to the present embodiment is physically aggregated and maintains a certain shape.

The green body may have any shape suitable for the application. The green body may have a shape of one or more selected from the group consisting of a cube, a rectangular parallelepiped, a polyhedron, a columnar shape, a cylindrical shape, a disk shape, a spherical shape and an approximately spherical shape and, in consideration of thermal shrinkage due to calcination, may also have a shape similar to the shape of a desired calcined body, for example, a discoidal shape used for CAD/CAM processing.

The green body may have a measured density of 2.75 g/cm$^3$ or more or 3.10 g/cm$^3$ or more, and 3.50 g/cm$^3$ or less or 3.40 g/cm$^3$ or less. Such a measured density corresponds to a relative density in the range of 45% to 58%.

The measured density is the density [g/cm$^3$] determined from the mass measured by mass measurement against the volume obtained from dimensions measured by dimensional measurement.

The green body may be produced by any forming method that can form the powder composition according to the present embodiment into a green compact. The forming method is, for example, one or more selected from the group consisting of uniaxial pressing, cold isostatic pressing (hereinafter also referred to as "CIP"), slip casting, sheet forming, slurry casting and injection molding. For convenience, the forming method is preferably at least one of slip casting, injection molding, uniaxial pressing and CIP, at least one of uniaxial pressing and CIP or uniaxial pressing followed by CIP. The pressure of the uniaxial pressing may be 15 MPa or more and 150 MPa or less, and the pressure of CIP may be 90 MPa or more and 400 MPa or less. The density of the resulting green body tends to increase with the forming pressure.

In the calcination step, the green body is calcined to produce a calcined body. Unlike green bodies (green compacts), a calcined body is composed of fused particles. The fused particles have a structure in the initial stage of sintering, and the calcined body has a structure in which particles form necking with each other in a state where the shape of the powder particles of the powder composition according to the present embodiment is partly maintained. Thus, the calcined body has mechanical characteristics suitable for machining.

The calcination in the calcination step may be heat treatment at a temperature lower than the sintering temperature of zirconia. In the present embodiment, calcined bodies with similar processability can be produced even when green bodies of powder compositions with different compositions are subjected to heat treatment under the same conditions. Thus, green bodies with different compositions can be simultaneously heat-treated to produce calcined bodies, and this further improves the production efficiency of calcined bodies. Furthermore, from the perspective of the production efficiency of calcined bodies, calcined bodies are preferably produced by firing at atmospheric pressure. The calcination conditions include the following conditions and may be appropriately set according to the amount of green body to be calcined and the characteristics of a calcination furnace to be used.

Calcination atmosphere: oxidizing atmosphere, preferably air atmosphere
Calcination temperature: 900°C or more, 950°C or more or 1000°C or more, and less than 1200°C, 1150°C or less or 1100°C or less
Calcination time: 0.5 hours or more, 1 hour or more or 2 hours or more, and 9 hours or less, 6 hours or less or 4 hours or less

In the present embodiment, the "firing at atmospheric pressure" is a method of heating an object to be treated without applying an external force to the object during heat treatment and, in particular, is a method of heating an object to be treated at a temperature lower than the sintering temperature without applying an external force to the object during heat treatment in the calcination step.

A calcined body (hereinafter also referred to as a "calcined body according to the present embodiment") is prepared by the calcination step, the calcined body containing fused particles of a transition metal compound other than zirconium and hafnium, zirconia in which two or more lanthanoid rare-earth elements are dissolved, and zirconia stabilized only by one or more selected from the group consisting of yttrium, calcium and magnesium, wherein the transition metal element other than zirconium and hafnium constitutes 1500 ppm or less.

The calcined body according to the present embodiment is preferably in a state where a coloring metal element is uniformly dispersed or dissolved in zirconia. The coloring metal element may be partly dissolved in zirconia. The state where the coloring metal element is uniformly dispersed in zirconia may be the state where there is no agglomerate of a compound containing the coloring metal element with a particle size of 0.5 $\mu$m or more.

The calcined body may have a hardness suitable for CAD/CAM machining, for example, a Vickers hardness in the range of 25 to 150 HV (= kgf/mm$^2$). The calcined body according to the present embodiment preferably has a Vickers hardness of 30 HV or more or 35 HV or more, and 70 HV or less, 60 HV or less or 50 HV or less.

In particular, the calcined bodies according to the present embodiment produced under the same production conditions (forming conditions and calcination conditions) preferably have almost the same Vickers hardness with the difference in Vickers hardness of 12 HV or less, 7 HV or less or 5 HV or less. This tends to result in calcined bodies with similar processability under the same processing conditions. The calcined bodies according to the present embodiment preferably have a small difference in Vickers hardness (hereinafter also referred to as a "hardness difference") resulting from the difference in composition, and the difference in Vickers hardness between the calcined bodies resulting from the difference in composition may be 0 HV or more, more than 0 HV, 1 HV or more or 2 HV or more.

A measure of the hardness difference may be a value obtained as an absolute difference (hereinafter also referred to as a "hardness difference (A2/C4)") by measuring the Vickers hardness of calcined bodies produced by forming and calcining under the following conditions a powder composition with a composition capable of producing a sintered body with a color tone corresponding to A2 or C4 in the Vita classical shade.

| (Forming conditions) CIP treatment | Forming method: uniaxial pressing and |
| | Uniaxial pressing pressure: 49 $\pm$ 3 |

(continued)

| MPa | |
|---|---|
| | CIP pressure: 196 ± 5 MPa |
| (Calcination conditions) atmospheric pressure | Calcination method: firing at |
| | Atmosphere: air atmosphere |
| | Firing time: 1000°C x 2 hours |
| | Heating rate: 50 ± 5°C/h |
| | Cooling rate: 300 ± 10°C/h |

Another measure of the hardness difference may be a value (hereinafter also referred to as a "hardness difference (A1)") obtained as an absolute difference in Vickers hardness between a calcined body (hereinafter also referred to as an "A1 calcined body") produced by forming and calcining under the above conditions a powder composition with a composition capable of producing a sintered body with a color tone corresponding to A1 in the Vita classical shade and a calcined body (hereinafter also referred to as an "X calcined body" according to the color tone of the calcined body; a calcined body with a color tone of B1 is also referred to as a "B1 calcined body") produced by forming and calcining under the above conditions a powder composition with a composition capable of producing a sintered body with a different color tone.

Although even a change in the yttrium content (the amount of stabilizing element) has little influence on the Vickers hardness values of the A1 calcined body and the X calcined body, the yttrium content of a calcined body used to evaluate the hardness difference (A1) may be 2.8% by mole or more and 6.0% by mole or less, 4.0% by mole or more and 4.5% by mole or less or 4.2% by mole.

More specifically, a calcined body used to evaluate the hardness difference (A1) may be a calcined body produced by filling a mold with a powder composition with an yttrium content of 4.244% by mole, an erbium content of 0.053% by mole, a terbium content of 0.002% by mole and a cobalt content of 15 ppm and titanium content of 75 ppm with the remainder being Y-stabilized $ZrO_2$, performing uniaxial pressing at a pressure of 49 MPa and then CIP treatment at a pressure of 196 MPa to produce a green body and heat-treating the green body in an air atmosphere at a calcination temperature of 1000°C for a calcination time of 2 hours.

The hardness difference (A1) is preferably small, more preferably 11 HV or less, 9 HV or less, 7 HV or less or 5 HV or less. This tends to result in calcined bodies with similar processability under the same processing conditions. The calcined body according to the present embodiment may have a hardness difference (A1) of 0 HV or more, more than 0 HV, 1 HV or more or 2 HV or more.

The hardness difference (A1) may be the absolute difference between the Vickers hardness of an A2, A3, A3.5, A4, B1, B2, B3, B4, C1, C2, C3, C4, D2, D3 or D4 calcined body and the Vickers hardness of the A1 calcined body.

In the present embodiment, the "Vickers hardness" can be measured with a typical Vickers tester (for example, Q30A, manufactured by Qness) equipped with a square pyramidal diamond indenter. In the measurement, the indenter is statically pressed onto the surface of a measurement sample, and the diagonal length of an indentation mark formed on the surface of the measurement sample is visually measured. The Vickers hardness can be calculated from the diagonal length using the following formula.

$$Hv = F/\{d^2/2\sin(\alpha/2)\}$$

In this formula, Hv denotes the Vickers hardness (HV), F denotes the measurement load (1 kgf), d denotes the diagonal length of an indentation mark (mm), and $\alpha$ denotes the angle between opposite faces of the indenter (136 degrees).

The measurement conditions for the Vickers hardness may be as follows:

Measurement sample: a disk shape with a thickness of 3.0 ± 0.5 mm
Measurement load: 1 kgf
As pretreatment before the measurement, the measurement surface of the measurement sample is polished with #800 waterproof abrasive paper to remove asperities exceeding 0.1 mm.

The calcined body according to the present embodiment may have a measured density of 2.75 $g/cm^3$ or more or 3.10 $g/cm^3$ or more, and 3.50 $g/cm^3$ or less or 3.40 $g/cm^3$ or less. Such a measured density corresponds to a relative density in the range of 45% to 58%. The calcined body may have a measured density similar to the measured density of the green body due to little densification caused by thermal shrinkage.

At least one of the powder composition according to the present embodiment and the calcined body according to the present embodiment can be used to produce a sintered body.

A sintered body is produced by a method for producing a sintered body including the step of sintering at least one of the powder composition according to the present embodiment and the calcined body according to the present embodiment. When a sintered body is directly produced from the powder composition according to the present embodiment, the powder composition may be formed into a green body and then sintered.

The sintering method may be a known sintering method, for example, one or more selected from the group consisting of pressureless sintering, pressure sintering and vacuum sintering. The sintering method is preferably a method suitable for the production of a dental prosthetic material and, more specifically, may be a sintering method not including pressure sintering or vacuum sintering, a sintering method including at least pressureless sintering, or pressureless sintering alone. Even when the sintering method is pressureless sintering alone, a zirconia sintered body suitable for a dental prosthesis can be produced as a so-called pressureless sintered body from at least one of the powder composition according to the present embodiment and the calcined body according to the present embodiment (hereinafter also referred to as "the powder composition according to the present embodiment or the like"). In the present embodiment, "pressureless sintering" is a method of sintering an object to be sintered by heating without applying an external force to the object during sintering.

The sintering conditions for pressureless sintering may be the following conditions and may be adjusted for the amounts of the green body, the calcined body and the like to be sintered and the characteristics of a sintering furnace.

Sintering temperature: 1200°C or more, 1300°C or more, 1400°C or more, 1430°C or more, 1450°C or more or 1500°C or more, and 1650°C or less, 1580°C or less or 1560°C or less
Heating rate: 50°C/h or more, 100°C/h or more, 150°C/h or more, and 500°C/min or less or 300°C/min or less
Sintering time: 0.1 hour or more, 0.5 hours or more or 1 hour or more, and 5 hours or less, 3 hours or less or 2 hours or less
Sintering atmosphere: at least one of an oxygen atmosphere and an air atmosphere, preferably an air atmosphere

The time required for the entire sintering process from the start of temperature rise to the end of temperature drop may be any time of 10 minutes or more and 10 hours or less. Furthermore, the green body (or calcined body) may be put into a firing furnace heated in advance, and then sintered. In the present embodiment, the air atmosphere is an atmosphere composed mainly of nitrogen and oxygen and with an oxygen concentration in the range of approximately 18% to 23% by volume.

A sintered body (hereinafter also referred to as a "sintered body according to the present embodiment") is produced by sintering at least one of the powder composition according to the present embodiment and the calcined body according to the present embodiment, wherein the sintered body is composed of crystal grains of zirconia in which a lanthanoid rare-earth element is dissolved and crystal grains of zirconia in which a transition metal element other than zirconium and hafnium is dissolved and that is stabilized only by one or more selected from the group consisting of yttrium, calcium and magnesium, wherein the zirconia in which the lanthanoid rare-earth element is dissolved contains two or more types of zirconia in which a different lanthanoid rare-earth element is dissolved, and the transition metal element content is 1500 ppm or less.

The sintered body according to the present embodiment preferably has a color tone similar to that of the dental color chart and more preferably has a color tone of A1, A2, A3, A3.5, A4, B1, B2, B3, B4, C1, C2, C3, C4, D2, D3 or D4 in the Vita classical shade.

A typical color tone in the present embodiment may be a color tone of the $L^*a^*b^*$ color system shown in Table 1. Table 2 shows examples of preferred color tones of the $L^*a^*b^*$ color system. However, the visible color tone of a sintered body varies with the transparency/translucency, and even sintered bodies belonging to the same color tone group may have different color tones.

[Table 1]

| Color tone group | L* | a* | b* | Color tone group | L* | a* | b* |
|---|---|---|---|---|---|---|---|
| A1 | 87-93 | -2-2 | 11-19 | C1 | 83-89 | -2-4 | 8-16 |
| A2 | 85-91 | 1-5 | 14-22 | C2 | 79-85 | 0-6 | 14-22 |
| A3 | 82-88 | 1-6 | 20-28 | C3 | 76-82 | 0-6 | 16-24 |
| A3.5 | 80-86 | 2-8 | 22-30 | C4 | 70-76 | 2-8 | 16-24 |
| A4 | 72-78 | 4-10 | 18-26 | D2 | 78-84 | 1-7 | 15-23 |

(continued)

| Color tone group | L* | a* | b* | Color tone group | L* | a* | b* |
|---|---|---|---|---|---|---|---|
| B1 | 87-93 | -4-2 | 14-22 | D3 | 79-85 | 3-9 | 21-29 |
| B2 | 85-91 | -2-4 | 16-24 | D4 | 80-86 | 0-6 | 19-27 |
| B3 | 82-88 | 0-6 | 17-25 | | | | |
| B4 | 80-86 | 1-7 | 24-32 | | | | |

[Table 2]

| Color tone group | L* | a* | b* | Color tone group | L* | a* | b* |
|---|---|---|---|---|---|---|---|
| A1 | 88.0-92.0 | -2.0-1.7 | 12.0-15.4 | C1 | 84.0-87.9 | -2.0-4.0 | 9.0-14.9 |
| A2 | 86.1-90.0 | 1.8-5.0 | 15.0-19.5 | C2 | 80.0-83.9 | 0-7.0 | 15.0-17.4 |
| A3 | 84.5-87.0 | 0-7.0 | 22.5-27.0 | C3 | 77.0-79.8 | 0-6.0 | 17.0-23.0 |
| A3.5 | 82.0-83.9 | 2.0-8.0 | 24.0-26.0 | C4 | 71.0-75.0 | 2.0-6.0 | 17.0-23.0 |
| A4 | 73.0-77.0 | 6.1-10.0 | 19.0-25.0 | D2 | 79.9-81.9 | 0-7.0 | 17.5-22.0 |
| B1 | 89.1-92.0 | -4.0-1.7 | 15.5-21.0 | D3 | 80.0-81.9 | 3.0-9.0 | 22.1-26.0 |
| B2 | 86.1-89.0 | -2.0-1.7 | 18.0-22.4 | D4 | 82.0-83.9 | 0-7.0 | 20.0-23.9 |
| B3 | 84.0-86.0 | 0-7.0 | 16.0-21.9 | | | | |
| B4 | 81.0-84.4 | 1.0-7.0 | 26.1-31.0 | | | | |

In the present embodiment, the color tone of a sintered body is a value measured in an SCI mode using a typical spectrophotometer (for example, CM-700d, manufactured by Konica Minolta, Inc.), a D65 light source as a light source and a white calibration plate as a background. A measurement sample may be a disk-shaped sintered body with a thickness of 1 ± 0.02 mm and a surface roughness Ra of 0.02 μm or less on both surfaces thereof.

The sintered body according to the present embodiment preferably has transparency/translucency, which may vary with the color tone, suitable for a dental prosthetic material. Such transparency/translucency is, for example, a total light transmittance of 15% or more, 20% or more, 25% or more or 30% or more, and 47% or less, 45% or less, 42% or less or 39% or less at a sample thickness of 1 mm (or 1 ± 0.1 mm) with respect to the D65 light source.

The total light transmittance is measured with a typical haze meter (for example, NDH4000, manufactured by Nippon Denshoku Industries Co., Ltd.) by a method according to JIS K 7361-1.

In the present embodiment, the sintered body preferably has a strength suitable for a dental prosthetic material. Such a strength is preferably a three-point bending strength of 800 MPa or more or 850 MPa or more, and 1200 MPa or less, 1000 MPa or less or 900 MPa or less.

In the present embodiment, the three-point bending strength is a value measured by a method according to JIS R 1601. A measurement sample may have a pillar shape with a width of 4 mm, a thickness of 3 mm and a length of 45 mm and may be subjected to measurement at a span of 30 mm under a load applied in the horizontal direction of the measurement sample.

The sintered body according to the present embodiment can be applied to a known use of a zirconia sintered body and can be particularly used as a dental material or a dental prosthesis. Examples of the dental prosthesis include crowns, bridges, inlays, onlays and veneers.

EXAMPLES

The present embodiment is described in the following examples. However, the present embodiment is not limited to these examples.

(Crystalline Phase and Monoclinic Ratio)

The crystalline phase of a powder composition was identified by XRD measurement under the following conditions using an X-ray diffractometer (apparatus name: Ultima IV, manufactured by Rigaku Corporation).

Radiation source: CuKα radiation (λ = 0.15418 nm)
Measurement mode: continuous scanning
Scanning speed: 4 degrees/min
Measurement range: 2θ = 26 to 33 degrees
Accelerating voltage and electric current: 40 mA and 40 kV
Divergence height limiting slit: 10 mm
Divergence/incident slit: 1 degree
Light-receiving slit: open
Detector: semiconductor detector (D/teX Ultra)
Filter: Ni filter
Goniometer radius: 185 mm

The crystalline layer was identified by smoothing treatment and background subtraction treatment using an analysis program attached to an X-ray diffractometer (program name: integrated X-ray powder diffraction software PDXL Ver. 2.2, manufactured by Rigaku Corporation) and by profile fitting of an XRD pattern after the treatment using a split pseudo-Voigt function.

The monoclinic ratio was determined from an XRD pattern after the treatment using the following formula.

$$f_M = [1 - \{[It(111) + Ic(111)]/[Im(111) + Im(11\text{-}1) + It(111) + Ic(111)]" \times 100$$

(Crystallite Size)

The crystallite size of a powder composition was determined using the following formula in a main XRD peak of an XRD pattern obtained by the method and treatment described in (Crystalline Phase and Monoclinic Ratio).

$$D = \kappa\lambda/\beta\cos\theta$$

(Density Measurement)

The densities of the green body and the calcined body were determined from the mass measured by mass measurement and the volume measured by dimensional measurement. In the dimensional measurement, the diameter of the upper end, the diameter of the lower end and the thickness of a disk-shaped sample were measured with a vernier caliper at four points, and the volume was determined from the average value of the thickness and the average value of the diameters of the upper and lower ends.

The density of a sintered body was measured by a method according to JIS R 1634.

(Average Granule Size)

The average granule size was defined as a granule size at 50% by mass in a cumulative granule size curve obtained by plotting the granule size and the mass ratio, which are obtained by a mechanical sieving method using a Ro-Tap sieve shaker (apparatus name: sieve shaker S-1, manufactured by Teraoka) under the following conditions.

Shaking frequency: 300 rpm
Shaking width: 25 mm
Standard number of blows of hammer: 150 rpm
Shaking time: 30 minutes

In the mechanical sieving method, sieves with apertures of 125 μm, 106 μm, 90 μm, 75 μm, 63 μm, 45 μm, 38 μm and 25 μm were stacked in this order in accordance with JIS Z 8801. The cumulative granule size curve was obtained by plotting the granule size and the mass ratio. The granule size of a powder composition remaining on each sieve after shaking was regarded as the granule size equivalent to the opening size of a sieve just above the sieve.

(Vickers Hardness)

The Vickers hardness is measured with a Vickers tester (apparatus name: Q30A, manufactured by Qness). Under the following conditions, an indenter is statically pressed onto the surface of a measurement sample, and the diagonal length of an indentation mark formed on the surface of the measurement sample is visually measured. The Vickers hardness was determined from the diagonal length using the formula described above.

Measurement sample: a disk shape with a thickness of 3.0 ± 0.5 mm
Measurement load: 1 kgf
A measurement sample was a calcined body, the measurement surface of which was polished by 0.1 mm with #800 waterproof abrasive paper before the measurement.

(Hardness Difference (A2/C4))

The hardness difference (A2/C4) was the absolute difference in Vickers hardness of calcined bodies produced by forming and calcining under the following conditions a powder composition with a composition capable of producing a sintered body with a color tone corresponding to A2 or C4 in the Vita classical shade.

| (Forming conditions) | Forming method: uniaxial pressing and |
| --- | --- |
| CIP treatment | |
| | Uniaxial pressing pressure: 49 ± 3 |
| MPa | |
| | CIP pressure: 196 ± 5 MPa |
| (Calcination conditions) atmospheric pressure | Calcination method: firing at |
| | Atmosphere: air atmosphere |
| | Firing time: 1000°C x 2 hours |
| | Heating rate: 50 ± 5°C/h |
| | Cooling rate: 300 ± 10°C/h |

(Hardness Difference (A1))

The hardness difference (A1) was the absolute difference between the Vickers hardness of an A2, A3, A3.5, A4, B1, B2, B3, B4, C1, C2, C3, C4, D2, D3 or D4 calcined body and the Vickers hardness of the A1 calcined body. A calcined body of Example 3 was used as the A1 calcined body.

(Total Light Transmittance)

The total light transmittance was measured by a method according to JIS K 7361-1 using a haze meter (apparatus name: NDH4000, manufactured by Nippon Denshoku Industries Co., Ltd.) as a measuring apparatus. A D65 light source was used as a light source.
A measurement sample was a disk-shaped sintered body sample with a diameter of 25 mm and a thickness of 1 mm, both surfaces of which were polished to a surface roughness Ra of 0.02 μm or less.

(Three-Point Bending Strength)

The three-point bending strength was measured by a method according to JIS R 1601. A measurement sample had a pillar shape with a width of 4 mm, a thickness of 3 mm and a length of 45 mm. The measurement was performed at a span of 30 mm under a load applied in the horizontal direction of the measurement sample.

(Color Tone)

The color tone was measured in an SCI mode using a spectrophotometer (apparatus name: CM-700d, manufactured by Konica Minolta, Inc.) and a D65 light source. The measurement was so-called white background measurement using a white calibration plate as a background. A measurement sample was a disk-shaped sintered body with a thickness of 1 mm, both surfaces of which were polished to a surface roughness Ra of 0.02 $\mu$m or less.

EXAMPLE 1

(Yttrium-Stabilized Zirconia Powder)

Yttrium chloride was added at an yttrium concentration of 4.3% by mole in terms of $Y_2O_3$ to zirconia sol prepared by hydrolyzing an aqueous solution of zirconium oxychloride. The zirconia sol was dried in air at 180°C and was fired at 1160°C for 2 hours. The firing was followed by drying in air at 110°C. 199.9 g of the fired product after drying, 0.1 g of an $\alpha$-alumina powder and pure water were mixed in a ball mill to prepare a slurry containing an yttrium-stabilized zirconia powder. The slurry was partially taken and was dried in air at 110°C to prepare an yttrium-stabilized zirconia powder, which was found to have a BET specific surface area of 10.1 m$^2$/g and an average particle size of 0.45 $\mu$m. An acrylic acid binder was added to and mixed with the slurry such that the mass ratio of the binder to the powder in the slurry was 3% by mass. The slurry was spray-dried in air at 180°C to prepare a granular powder (hereinafter also referred to as an "Y(4.3)-stabilized $ZrO_2$ granular powder") containing 3% by mass of the acrylic acid binder and 0.05% by mass of alumina with the remainder being zirconia stabilized by 4.3% by mole of yttrium. The granular powder had an average granule size of 44 pm.

(Yttrium-Stabilized Terbium Solid Solution Zirconia Powder)

A slurry containing an yttrium-stabilized terbium solid solution zirconia powder was prepared in the same manner as the yttrium-stabilized zirconia powder except that yttrium chloride was added to the zirconia sol at an yttrium concentration of 4.3% by mole in terms of $Y_2O_3$ and terbium oxide (III, IV) was added to the zirconia sol at a terbium concentration of 0.04% by mole in terms of $Tb_4O_7$. The yttrium-stabilized terbium solid solution zirconia powder had a BET specific surface area of 10.2 m$^2$/g and an average particle size of 0.44 pm.

199.9 g of the yttrium-stabilized terbium solid solution zirconia powder, 0.1 g of an $\alpha$-alumina powder and pure water were mixed in a ball mill to prepare a slurry. An acrylic acid binder was added to and mixed with the slurry such that the mass ratio of the binder to the powder in the slurry was 3% by mass. The slurry was spray-dried in air at 180°C to prepare a granular powder (hereinafter also referred to as an "Y(4.3)-stabilized Tb solid solution $ZrO_2$ granular powder") containing 3% by mass of the acrylic acid binder and 0.05% by mass of alumina with the remainder being zirconia that was stabilized by 4.3% by mole of yttrium and in which 0.04% by mole of terbium was dissolved. The granular powder had an average granule size of 43 pm.

(Erbium Solid Solution Zirconia Powder)

A slurry containing an erbium solid solution zirconia powder was prepared in the same manner as the yttrium-stabilized zirconia powder except that in place of yttrium chloride erbium oxide was added to the zirconia sol at an erbium concentration of 4.4% by mole in terms of $Er_2O_3$. The erbium solid solution zirconia powder had a BET specific surface area of 9.8 m$^2$/g and an average particle size of 0.45 $\mu$m.

199.9 g of the erbium solid solution zirconia powder, 0.1 g of an $\alpha$-alumina powder and pure water were mixed in a ball mill to prepare a slurry. An acrylic acid binder was added to and mixed with the slurry such that the mass ratio of the binder to the powder in the slurry was 3% by mass. The slurry was spray-dried in air at 180°C to prepare a granular powder (hereinafter also referred to as an "Er(4.4) solid solution $ZrO_2$ granular powder") containing 3% by mass of the acrylic acid binder and 0.05% by mass of alumina with the remainder being zirconia in which 4.4% by mole of erbium was dissolved. The granular powder had an average granule size of 42 pm.

(Granular Powder Containing Cobalt Oxide and Titanium Oxide)

A cobalt oxide powder ($Co_3O_4$) and a titanium oxide powder ($TiO_2$) were used as coloring metal powders. The yttrium-stabilized zirconia powder prepared in the present example was partially taken and mixed with an $\alpha$-alumina powder, a cobalt oxide powder, a titanium oxide powder and pure water in a ball mill to prepare a slurry. An acrylic acid binder was added to and mixed with the slurry such that the mass ratio of the binder to the powder in the slurry was 3% by mass. The slurry was spray-dried in air at 180°C to prepare a granular powder (hereinafter also referred to as a "Co-Ti-Y(4.3)-stabilized $ZrO_2$ granular powder") containing 3% by mass of the acrylic acid binder, 0.05% by mass of alumina, 0.06% by mass of tricobalt tetraoxide and 0.3% by mass of titanium oxide with the remainder being zirconia stabilized by 4.3% by mole of yttrium. The granular powder had an average granule size of 45 pm.

(Powder Composition)

A 200-mL polypropylene vessel was filled with the Y(4.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder prepared in the present example at a mass ratio of 85.6 (85.55) :6.5 (6.45):3.5 (3.50) :4.5 (4.50). The mixture was dry-mixed by stirring to prepare a powder composition according to the present example with the following composition and with a coloring metal element content of 144 ppm.

Yttrium content: 4.154% by mole
Erbium content: 0.146% by mole
Terbium content: 0.003% by mole
Cobalt content: 24 ppm
Titanium content: 120 ppm
Alumina content: 0.05% by mass
Zirconia content: remainder

The prepared powder composition had a BET specific surface area of 10.1 $m^2$/g, an average particle size of 0.45 $\mu$m, a crystallite size of 380 nm, a monoclinic ratio of 6% and an average granule size of 44 $\mu$m. The Y(4.3)-stabilized $ZrO_2$ granular powder, the Er(4.4) solid solution $ZrO_2$ granular powder and the Y(4.3)-stabilized Tb solid solution $ZrO_2$ granular powder had a BET difference of 0.4 $m^2$/g and a particle size difference of 0.01 pm.

(Green Body, Calcined Body and Sintered Body)

5.5 g of the prepared powder composition filled in a mold with a diameter of 25 mm was subjected to uniaxial pressing at a pressure of 49 MPa and then CIP treatment at a pressure of 196 MPa to prepare a green body (green compact).
The prepared green body was calcined under the following conditions to prepare a calcined body according to the present example.

Calcination temperature: 1000°C
Calcination time: 2 hours
Heating rate: 50°C/h
Calcination atmosphere: air atmosphere
Cooling rate: 300°C/h

The calcined body according to the present example was sintered under the following conditions to prepare a sintered body according to the present example.

Sintering method: pressureless sintering
Sintering temperature: 1500°C
Sintering time: 2 hours
Heating rate: 600°C/h
Sintering atmosphere: air atmosphere

EXAMPLE 2

A powder composition according to the present example with the following composition and with a coloring metal element content of 900 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 22.5:46.0:6.5:25.0.

Yttrium content: 4.037% by mole
Erbium content: 0.273% by mole
Terbium content: 0.019% by mole
Cobalt content: 150 ppm
Titanium content: 750 ppm
Alumina content: 0.05% by mass

Zirconia content: remainder

The prepared powder composition had a BET specific surface area of 10.2 m$^2$/g, an average particle size of 0.45 $\mu$m, a crystallite size of 380 nm, a monoclinic ratio of 7% and an average granule size of 44 pm.

EXAMPLE 3

A powder composition according to the present example with the following composition and with a coloring metal element content of 90 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 91.7:4.5:1.3:2.5.

Yttrium content: 4.244% by mole
Erbium content: 0.053% by mole
Terbium content: 0.002% by mole
Cobalt content: 15 ppm
Titanium content: 75 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 7% and an average granule size of 44 pm.

EXAMPLE 4

A powder composition according to the present example with the following composition and with a coloring metal element content of 180 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 77.0:15.0:3.0:5.0.

Yttrium content: 4.176% by mole
Erbium content: 0.125% by mole
Terbium content: 0.006% by mole
Cobalt content: 30 ppm
Titanium content: 150 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 7% and an average granule size of 44 pm.

EXAMPLE 5

A powder composition according to the present example with the following composition and with a coloring metal element content of 270 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 64.9:22.5:5.1:7.5.

Yttrium content: 4.089% by mole
Erbium content: 0.215% by mole
Terbium content: 0.009% by mole
Cobalt content: 45 ppm
Titanium content: 225 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 7% and an average granule size of 44 pm.

EXAMPLE 6

A powder composition according to the present example with the following composition and with a coloring metal element content of 720 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 35.6:35.0:9.4:20.0.

Yttrium content: 3.917% by mole
Erbium content: 0.395% by mole
Terbium content: 0.014% by mole
Cobalt content: 120 ppm
Titanium content: 600 ppm
Alumina content: 0.05% by mass
Zirconia content: remainder

The prepared powder composition had a BET specific surface area of 10.1 m$^2$/g, an average particle size of 0.45 μm, a crystallite size of 375 angstroms, a monoclinic ratio of 7% and an average granule size of 44 pm.

EXAMPLE 7

A powder composition according to the present example with the following composition and with a coloring metal element content of 90 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 90.6:6.0:0.9:2.5.

Yttrium content: 4.262% by mole
Erbium content: 0.035% by mole
Terbium content: 0.002% by mole
Cobalt content: 15 ppm
Titanium content: 75 ppm
Alumina content: 0.05% by mass
Zirconia content: remainder

The prepared powder composition had a BET specific surface area of 10.1 m$^2$/g, an average particle size of 0.45 μm, a crystallite size of 370 angstroms, a monoclinic ratio of 7% and an average granule size of 44 μm.

EXAMPLE 8

A powder composition according to the present example with the following composition and with a coloring metal element content of 118 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 86.5:8.50:1.7:3.3.

Yttrium content: 4.227% by mole
Erbium content: 0.071% by mole
Terbium content: 0.003% by mole
Cobalt content: 20 ppm
Titanium content: 98 ppm
Alumina content: 0.05% by mass
Zirconia content: remainder

The prepared powder composition had a BET specific surface area of 10.1 m$^2$/g, an average particle size of 0.45 μm, a crystallite size of 370 angstroms, a monoclinic ratio of 7% and an average granule size of 44 μm.

EXAMPLE 9

A powder composition according to the present example with the following composition and with a coloring metal element content of 226 ppm was prepared in the same manner as in Example 1 except that the Y(4.3)-stabilized

ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 79.4:11.5:2.8:6.3.

Yttrium content: 4.182% by mole
Erbium content: 0.118% by mole
Terbium content: 0.005% by mole
Cobalt content: 38 ppm
Titanium content: 188 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 7% and an average granule size of 44 pm.

EXAMPLE 10

A powder composition according to the present example with the following composition and with a coloring metal element content of 270 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 64.5:25.0:3.0:7.5.

Yttrium content: 4.177% by mole
Erbium content: 0.125% by mole
Terbium content: 0.010% by mole
Cobalt content: 45 ppm
Titanium content: 225 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 7% and an average granule size of 44 pm.

EXAMPLE 11

A powder composition according to the present example with the following composition and with a coloring metal element content of 270 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 86.2:5.0:1.3:7.5.

Yttrium content: 4.245% by mole
Erbium content: 0.053% by mole
Terbium content: 0.002% by mole
Cobalt content: 45 ppm
Titanium content: 225 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 7% and an average granule size of 44 pm.

EXAMPLE 12

A powder composition according to the present example with the following composition and with a coloring metal element content of 406 ppm was prepared in the same manner as in Example 1 except that the Y(4.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 75.6:11.0:2.1:11.3.

Yttrium content: 4.211% by mole

Erbium content: 0.089% by mole
Terbium content: 0.004% by mole
Cobalt content: 68 ppm
Titanium content: 338 ppm
Alumina content: 0.05% by mass
Zirconia content: remainder

The prepared powder composition had a BET specific surface area of 10.1 $m^2/g$, an average particle size of 0.45 $\mu$m, a crystallite size of 370 angstroms, a monoclinic ratio of 7% and an average granule size of 44 pm.

EXAMPLE 13

A powder composition according to the present example with the following composition and with a coloring metal element content of 630 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 61.2:20.0:1.3:17.5.

Yttrium content: 4.248% by mole
Erbium content: 0.053% by mole
Terbium content: 0.008% by mole
Cobalt content: 105 ppm
Titanium content: 525 ppm
Alumina content: 0.05% by mass
Zirconia content: remainder

The prepared powder composition had a BET specific surface area of 10.1 $m^2/g$, an average particle size of 0.45 $\mu$m, a crystallite size of 370 angstroms, a monoclinic ratio of 7% and an average granule size of 44 pm.

EXAMPLE 14

A powder composition according to the present example with the following composition and with a coloring metal element content of 450 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 69.5:15.0:3.0:12.5.

Yttrium content: 4.177% by mole
Erbium content: 0.125% by mole
Terbium content: 0.006% by mole
Cobalt content: 75 ppm
Titanium content: 375 ppm
Alumina content: 0.05% by mass
Zirconia content: remainder

The prepared powder composition had a BET specific surface area of 10.1 $m^2/g$, an average particle size of 0.45 $\mu$m, a crystallite size of 370 angstroms, a monoclinic ratio of 7% and an average granule size of 44 pm.

EXAMPLE 15

A powder composition according to the present example with the following composition and with a coloring metal element content of 316 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 63.6:22.5:5.1:8.8.

Yttrium content: 4.089% by mole
Erbium content: 0.215% by mole
Terbium content: 0.009% by mole
Cobalt content: 53 ppm
Titanium content: 263 ppm

Alumina content: 0.05% by mass
Zirconia content: remainder

The prepared powder composition had a BET specific surface area of 10.1 m²/g, an average particle size of 0.45 μm, a crystallite size of 370 angstroms, a monoclinic ratio of 7% and an average granule size of 44 pm.

EXAMPLE 16

A powder composition according to the present example with the following composition and with a coloring metal element content of 334 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 71.5:17.5:1.7:9.3.

Yttrium content: 4.229% by mole
Erbium content: 0.071% by mole
Terbium content: 0.007% by mole
Cobalt content: 56 ppm
Titanium content: 278 ppm
Alumina content: 0.05% by mass
Zirconia content: remainder

The prepared powder composition had a BET specific surface area of 10.1 m²/g, an average particle size of 0.45 μm, a crystallite size of 370 angstroms, a monoclinic ratio of 7% and an average granule size of 44 pm.

EXAMPLE 17

(Yttrium-Stabilized Zirconia Powder)

A granular powder (hereinafter also referred to as an "Y(2.9)-stabilized $ZrO_2$ granular powder 1") containing 3% by mass of the acrylic acid binder and 0.05% by mass of alumina with the remainder being zirconia stabilized by 2.9% by mole of yttrium was prepared in the same manner as the yttrium-stabilized zirconia powder according to Example 1 except that yttrium chloride was added to the zirconia sol at an yttrium concentration of 2.9% by mole in terms of $Y_2O_3$ and the zirconia sol was fired at 1100°C for 2 hours. In the same manner as in Example 1, the yttrium-stabilized zirconia powder was found to have a BET specific surface area of 13.0 m²/g and an average particle size of 0.45 μm. The granular powder had an average granule size of 44 pm.

(Powder Composition)

A powder composition according to the present example with the following composition and with a coloring metal element content of 118 ppm was prepared in the same manner as in Example 1 except that the Y(2.9)-stabilized $ZrO_2$ granular powder 1, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 87.3:7.5:2.0:3.2.

Yttrium content: 3.025% by mole
Erbium content: 0.083% by mole
Terbium content: 0.003% by mole
Cobalt content: 20 ppm
Titanium content: 98 ppm
Alumina content: 0.05% by mass
Zirconia content: remainder

The prepared powder composition had a BET specific surface area of 12.6 m²/g, an average particle size of 0.45 μm, a crystallite size of 360 angstroms, a monoclinic ratio of 31% and an average granule size of 44 pm.

EXAMPLE 18

A powder composition according to the present example with the following composition and with a coloring metal element content of 630 ppm was prepared in the same manner as in Example 1 except that the Y(2.9)-stabilized

ZrO$_2$ granular powder 1, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 61.2:20.0:1.3:17.5.

Yttrium content: 3.410% by mole
Erbium content: 0.053% by mole
Terbium content: 0.008% by mole
Cobalt content: 105 ppm
Titanium content: 525 ppm
Alumina content: 0.05% by mass
Zirconia content: remainder

The prepared powder composition had a BET specific surface area of 11.8 m$^2$/g, an average particle size of 0.45 μm, a crystallite size of 370 angstroms, a monoclinic ratio of 24% and an average granule size of 44 pm.

EXAMPLE 19

A powder composition according to the present example with the following composition and with a coloring metal element content of 960 ppm was prepared in the same manner as in Example 1 except that the Y(2.9)-stabilized ZrO$_2$ granular powder 1, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 24.0:45.0:4.3:26.7.

Yttrium content: 3.844% by mole
Erbium content: 0.178% by mole
Terbium content: 0.018% by mole
Cobalt content: 160 ppm
Titanium content: 800 ppm
Alumina content: 0.05% by mass
Zirconia content: remainder

The prepared powder composition had a BET specific surface area of 10.6 m$^2$/g, an average particle size of 0.45 μm, a crystallite size of 370 angstroms, a monoclinic ratio of 13% and an average granule size of 44 pm.

EXAMPLE 20

(Yttrium-Stabilized Zirconia Powder)

A granular powder (hereinafter also referred to as an "Y(2.9)-stabilized ZrO$_2$ granular powder 2") containing 3% by mass of the acrylic acid binder and 0.05% by mass of alumina with the remainder being zirconia stabilized by 2.9% by mole of yttrium was prepared in the same manner as the yttrium-stabilized zirconia powder according to Example 1 except that yttrium chloride was added to the zirconia sol at an yttrium concentration of 2.9% by mole in terms of Y$_2$O$_3$ and the zirconia sol was fired at 1175°C for 2 hours. In the same manner as in Example 1, the yttrium-stabilized zirconia powder was found to have a BET specific surface area of 10.1 m$^2$/g and an average particle size of 0.45 μm. The granular powder had an average granule size of 44 μm.

(Powder Composition)

A powder composition according to the present example with the following composition and with a coloring metal element content of 118 ppm was prepared in the same manner as in Example 1 except that the Y(2.9)-stabilized ZrO$_2$ granular powder 2, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 87.3:7.5:2.0:3.2.

Yttrium content: 3.025% by mole
Erbium content: 0.083% by mole
Terbium content: 0.003% by mole
Cobalt content: 20 ppm
Titanium content: 98 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 30% and an average granule size of 44 pm.

EXAMPLE 21

A powder composition according to the present example with the following composition and with a coloring metal element content of 630 ppm was prepared in the same manner as in Example 1 except that the Y(2.9)-stabilized ZrO$_2$ granular powder 2, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 61.2:20.0:1.3:17.5.

Yttrium content: 3.410% by mole
Erbium content: 0.053% by mole
Terbium content: 0.008% by mole
Cobalt content: 105 ppm
Titanium content: 525 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 20% and an average granule size of 44 pm.

EXAMPLE 22

A powder composition according to the present example with the following composition and with a coloring metal element content of 960 ppm was prepared in the same manner as in Example 1 except that the Y(2.9)-stabilized ZrO$_2$ granular powder 2, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 24.0:45.0:4.3:26.7.

Yttrium content: 3.844% by mole
Erbium content: 0.178% by mole
Terbium content: 0.018% by mole
Cobalt content: 160 ppm
Titanium content: 800 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 13% and an average granule size of 44 pm.

EXAMPLE 23

(Yttrium-Stabilized Zirconia Powder)

A powder of zirconia stabilized by 5.3% by mole of yttrium was prepared in the same manner as the yttrium-stabilized zirconia powder according to Example 1 except that yttrium chloride was added to the zirconia sol at an yttrium concentration of 5.3% by mole in terms of Y$_2$O$_3$ and the zirconia powder was fired at 1175°C for 2 hours. In the same manner as in Example 1, the yttrium-stabilized zirconia powder was found to have a BET specific surface area of 10.0 m$^2$/g and an average particle size of 0.45 $\mu$m. A granular powder (hereinafter also referred to as an "Y(5.3)-stabilized ZrO$_2$ granular powder") was prepared that contained the yttrium-stabilized zirconia powder, 3% by mass of the acrylic acid binder and 0.05% by mass of alumina with the remainder being zirconia stabilized by 5.3% by mole of yttrium. The granular powder had an average granule size of 44 $\mu$m.

(Powder Composition)

A powder composition according to the present example with the following composition and with a coloring metal element content of 198 ppm was prepared in the same manner as in Example 1 except that the Y(5.3)-stabilized ZrO$_2$ granular powder, the Y(4.3)-stabilized Tb solid solution ZrO$_2$ granular powder, the Er(4.4) solid solution ZrO$_2$ granular powder and the Co-Ti-Y(4.3)-stabilized ZrO$_2$ granular powder were mixed at a mass ratio of 74.9:16.5:3.1:5.5.

Yttrium content: 4.945% by mole
Erbium content: 0.130% by mole
Terbium content: 0.007% by mole
Cobalt content: 33 ppm
Titanium content: 165 ppm
Alumina content: 0.05% by mass
Zirconia content: remainder

The prepared powder composition had a BET specific surface area of 10.1 $m^2$/g, an average particle size of 0.45 $\mu$m, a crystallite size of 390 angstroms, a monoclinic ratio of 2% and an average granule size of 44 pm.

EXAMPLE 24

A powder composition according to the present example with the following composition and with a coloring metal element content of 648 ppm was prepared in the same manner as in Example 1 except that the Y(5.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 61.1:20.3:0.6:18.0.

Yttrium content: 4.905% by mole
Erbium content: 0.026% by mole
Terbium content: 0.008% by mole
Cobalt content: 108 ppm
Titanium content: 540 ppm
Alumina content: 0.05% by mass
Zirconia content: remainder

The prepared powder composition had a BET specific surface area of 10.1 $m^2$/g, an average particle size of 0.45 $\mu$m, a crystallite size of 380 angstroms, a monoclinic ratio of 3% and an average granule size of 44 pm.

EXAMPLE 25

A powder composition according to the present example with the following composition and with a coloring metal element content of 936 ppm was prepared in the same manner as in Example 1 except that the Y(5.3)-stabilized $ZrO_2$ granular powder, the Y(4.3)-stabilized Tb solid solution $ZrO_2$ granular powder, the Er(4.4) solid solution $ZrO_2$ granular powder and the Co-Ti-Y(4.3)-stabilized $ZrO_2$ granular powder were mixed at a mass ratio of 35.4:36.4:2.2:26.0.

Yttrium content: 4.577% by mole
Erbium content: 0.093% by mole
Terbium content: 0.015% by mole
Cobalt content: 156 ppm
Titanium content: 780 ppm
Alumina content: 0.05% by mass
Zirconia content: remainder

The prepared powder composition had a BET specific surface area of 10.1 $m^2$/g, an average particle size of 0.45 $\mu$m, a crystallite size of 380 angstroms, a monoclinic ratio of 5% and an average granule size of 44 pm.

EXAMPLE 26

(Titanium-Oxide-Containing Yttrium-Stabilized Neodymium Solid Solution Zirconia Powder)

A powder of zirconia that was stabilized by 1.6% by mole of yttrium and in which 1.5% by mole of neodymium was dissolved was prepared in the same manner as in Example 1 except that yttrium chloride was added to the zirconia sol at an yttrium concentration of 1.6% by mole in terms of $Y_2O_3$, neodymium oxide was added to the zirconia sol at a neodymium concentration of 1.5% by mole in terms of $Nd_2O_3$, and the zirconia sol was fired at 1120°C for 2 hours. In the same manner as in Example 1, the powder was found to have a BET specific surface area of 10.1 $m^2$/g and an average particle size of 0.45 $\mu$m. A granular powder (hereinafter also referred to as a "Ti-Y(1.6)-stabilized Nd solid solution $ZrO_2$ granular powder") containing 3% by mass of the acrylic acid binder, 0.05% by mass of alumina and 0.1% by mass of titanium oxide with the remainder being zirconia that was stabilized by 1.6% by mole of yttrium and in which 1.5% by mole of neodymium was dissolved was prepared in the same manner as the yttrium-stabilized zirconia powder according to Example 1 except that the prepared powder, an alumina powder, a titanium oxide powder and pure water were mixed in a ball mill. The granular powder had an average granule size of 44 μm.

(Powder Composition)

A powder composition according to the present example with the following composition and with a coloring metal element content of 15 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Ti-Y(1.6)-stabilized Nd solid solution $ZrO_2$ granular powder were mixed at a mass ratio of 87.9:7.5:3.1:1.5.

Yttrium content: 4.127% by mole
Erbium content: 0.132% by mole
Terbium content: 0.003% by mole
Neodymium content: 0.023% by mole
Titanium content: 15 ppm
Alumina content: 0.05% by mass
Zirconia content: remainder

The prepared powder composition had a BET specific surface area of 10.1 $m^2/g$, an average particle size of 0.45 μm, a crystallite size of 370 angstroms, a monoclinic ratio of 7% and an average granule size of 44 pm.

EXAMPLE 27

A powder composition according to the present example with the following composition and with a coloring metal element content of 189 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Ti-Y(1.6)-stabilized Nd solid solution $ZrO_2$ granular powder were mixed at a mass ratio of 39.5:38.4:3.2:18.9.

Yttrium content: 3.667% by mole
Erbium content: 0.135% by mole
Terbium content: 0.016% by mole
Neodymium content: 0.288% by mole
Titanium content: 189 ppm
Alumina content: 0.05% by mass
Zirconia content: remainder

The prepared powder composition had a BET specific surface area of 10.1 $m^2/g$, an average particle size of 0.45 μm, a crystallite size of 370 angstroms, a monoclinic ratio of 10% and an average granule size of 44 pm.

EXAMPLE 28

A powder composition according to the present example with the following composition and with a coloring metal element content of 323 ppm was prepared in the same manner as in Example 1 except that the Y(4.3)-stabilized $ZrO_2$ granular powder, the Y(4.3)-stabilized Tb solid solution $ZrO_2$ granular powder, the Er(4.4) solid solution $ZrO_2$ granular powder and the Ti-Y(1.6)-stabilized Nd solid solution $ZrO_2$ granular powder were mixed at a mass ratio of 6.4:58.3:3.0:32.3.

Yttrium content: 3.320% by mole
Erbium content: 0.126% by mole
Terbium content: 0.024% by mole
Neodymium content: 0.492% by mole
Titanium content: 323 ppm
Alumina content: 0.05% by mass
Zirconia content: remainder

The prepared powder composition had a BET specific surface area of 10.1 m²/g, an average particle size of 0.45 μm, a crystallite size of 370 angstroms, a monoclinic ratio of 12% and an average granule size of 44 pm.

EXAMPLE 29

(Yttrium-Stabilized Praseodymium Solid Solution Zirconia Powder)

A granular powder (hereinafter also referred to as an "Y(1.6)-stabilized Pr solid solution $ZrO_2$ granular powder") containing 3% by mass of the acrylic acid binder and 0.05% by mass of alumina with the remainder being zirconia that was stabilized by 1.6% by mole of yttrium and in which 0.76% by mole of praseodymium was dissolved was prepared in the same manner as the yttrium-stabilized zirconia powder according to Example 1 except that yttrium chloride was added to the zirconia sol at an yttrium concentration of 1.6% by mole in terms of $Y_2O_3$, praseodymium oxide was added to the zirconia sol at a praseodymium concentration of 0.76% by mole in terms of $Pr_6O_{11}$, and the zirconia sol was fired at 1120°C for 2 hours. In the same manner as in Example 1, the powder was found to have a BET specific surface area of 10.0 m²/g and an average particle size of 0.45 μm. The prepared powder, the granular powder, had an average granule size of 43 μm.

(Powder Composition)

A powder composition according to the present example with the following composition and with a coloring metal element content of 550 ppm was prepared in the same manner as in Example 1 except that the Y(4.3)-stabilized $ZrO_2$ granular powder, the Er(4.4) solid solution $ZrO_2$ granular powder, the Co-Ti-Y(4.3)-stabilized $ZrO_2$ granular powder and the Y(1.6)-stabilized Pr solid solution $ZrO_2$ granular powder were mixed at a mass ratio of 82.5:1.0:15.3:1.2.

Yttrium content: 4.225% by mole
Erbium content: 0.042% by mole
Praseodymium content: 0.012% by mole
Cobalt content: 92 ppm
Titanium content: 458 ppm
Alumina content: 0.05% by mass
Zirconia content: remainder

The prepared powder composition had a BET specific surface area of 10.1 m²/g, an average particle size of 0.45 μm, a crystallite size of 370 angstroms, a monoclinic ratio of 8% and an average granule size of 44 pm.

EXAMPLE 30

A powder composition according to the present example with the following composition and with a coloring metal element content of 550 ppm was prepared in the same manner as in Example 1 except that the Y(4.3)-stabilized $ZrO_2$ granular powder, the Er(4.4) solid solution $ZrO_2$ granular powder, the Co-Ti-Y(4.3)-stabilized $ZrO_2$ granular powder and the Y(1.6)-stabilized Pr solid solution $ZrO_2$ granular powder were mixed at a mass ratio of 81.8:0.9:15.3:2.0.

Yttrium content: 4.210% by mole
Erbium content: 0.036% by mole
Praseodymium content: 0.020% by mole
Cobalt content: 92 ppm
Titanium content: 458 ppm
Alumina content: 0.05% by mass
Zirconia content: remainder

The prepared powder composition had a BET specific surface area of 10.1 m²/g, an average particle size of 0.45 μm, a crystallite size of 370 angstroms, a monoclinic ratio of 9% and an average granule size of 44 μm.

EXAMPLE 31

A powder composition according to the present example with the following composition and with a coloring metal element content of 1079 ppm was prepared in the same manner as in Example 1 except that the Y(4.3)-stabilized $ZrO_2$ granular powder, the Er(4.4) solid solution $ZrO_2$ granular powder, the Co-Ti-Y(4.3)-stabilized $ZrO_2$ granular powder

and the Y(1.6)-stabilized Pr solid solution $ZrO_2$ granular powder were mixed at a mass ratio of 56.3:1.2:39.5:3.0.

Yttrium content: 4.173% by mole
Erbium content: 0.050% by mole
Praseodymium content: 0.030% by mole
Cobalt content: 229 ppm
Titanium content: 850 ppm
Alumina content: 0.05% by mass
Zirconia content: remainder

The prepared powder composition had a BET specific surface area of 10.1 m$^2$/g, an average particle size of 0.45 μm, a crystallite size of 370 angstroms, a monoclinic ratio of 10% and an average granule size of 44 μm.

(Green Body, Calcined Body and Sintered Body)

Green bodies (green compacts), calcined bodies and sintered bodies were prepared in the same manner as in Example 1 except that the powder compositions prepared in Examples 2 to 31 were used.

COMPARATIVE EXAMPLE 1

(Yttrium-Stabilized Zirconia Powder)

In the same manner as the yttrium-stabilized zirconia powder according to Example 1, a granular powder (an Y(4.3)-stabilized $ZrO_2$ granular powder) containing 0.05% by mass of alumina with the remainder being zirconia stabilized by 4.3% by mole of yttrium was prepared.

(Erbium Solid Solution Zirconia Powder)

In the same manner as the erbium solid solution zirconia powder according to Example 1, a granular powder (an Er(4.4) solid solution $ZrO_2$ granular powder) containing 0.05% by mass of alumina with the remainder being zirconia in which 4.4% by mole of erbium was dissolved was prepared.

(Cobalt Oxide Granular Powder)

A cobalt oxide powder ($Co_3O_4$) was used as the coloring metal powder. The yttrium-stabilized zirconia powder prepared in the present comparative example was partially taken and mixed with an α-alumina powder, cobalt oxide and pure water in a ball mill to prepare a slurry. An acrylic acid binder was added to and mixed with the slurry such that the mass ratio of the binder to the powder in the slurry was 3% by mass. The slurry was spray-dried in air at 180°C to prepare a granular powder (hereinafter also referred to as a "Co-Y(4.3)-stabilized $ZrO_2$ granular powder") containing 3% by mass of the acrylic acid binder, 0.05% by mass of alumina and 0.06% by mass of cobalt oxide with the remainder being zirconia stabilized by 4.3% by mole of yttrium. The prepared granular powder had an average granule size of 44 μm.

(Iron Oxide Granular Powder)

An iron oxide ($Fe_2O_3$) powder was used as the coloring metal powder. The yttrium-stabilized zirconia powder prepared in the present comparative example was partially taken and mixed with an α-alumina powder, iron oxide and pure water in a ball mill to prepare a slurry. An acrylic acid binder was added to and mixed with the slurry such that the mass ratio of the binder to the powder in the slurry was 3% by mass. The slurry was spray-dried in air at 180°C to prepare a granular powder (hereinafter also referred to as an "Fe-Y(4.3)-stabilized $ZrO_2$ granular powder") containing 3% by mass of the acrylic acid binder, 0.05% by mass of alumina and 0.2% by mass of iron oxide with the remainder being zirconia stabilized by 4.3% by mole of yttrium. The prepared granular powder had an average granule size of 46 pm.

(Powder Composition)

A 200-mL polypropylene vessel was filled with the Y(4.3)-stabilized $ZrO_2$ granular powder, the Er(4.4) solid solution $ZrO_2$ granular powder, the Co-Y(4.3)-stabilized $ZrO_2$ granular powder and the Fe-Y(4.3)-stabilized $ZrO_2$ granular powder at a mass ratio of 85.6 (85.55) :6.5 (6.45) :3.5 (3.50) :4.5 (4.50) . The mixture was dry-mixed by stirring to prepare a powder composition according to the present comparative example with the following composition and with a coloring metal element content of 700 ppm.

> Yttrium content: 4.166% by mole
> Erbium content: 0.126% by mole
> Iron content: 700 ppm
> Cobalt content: 0 ppm
> Alumina content: 0.05% by mass
> Zirconia content: remainder

The prepared powder composition had a BET specific surface area of 10.1 $m^2/g$, an average particle size of 0.45 $\mu$m, a crystallite size of 370 nm, a monoclinic ratio of 7% and an average granule size of 45 pm.

(Green Body, Calcined Body and Sintered Body)

A green body (green compact), a calcined body and a sintered body were prepared in the same manner as in Example 1 except that the prepared powder composition was used.

COMPARATIVE EXAMPLE 2

A powder composition according to the present comparative example with the following composition and with a coloring metal element content of 1372 ppm was prepared in the same manner as in Comparative Example 1 except that the Y(4.3)-stabilized $ZrO_2$ granular powder, the Er(4.4) solid solution $ZrO_2$ granular powder, the Co-Y(4.3)-stabilized $ZrO_2$ granular powder and the Fe-Y(4.3)-stabilized $ZrO_2$ granular powder were dry-mixed at a mass ratio of 19.7 (19.65):1.1 (1.08):15.3 (15.27):64.0 (64.00).

> Yttrium content: 4.257% by mole
> Erbium content: 0.042% by mole
> Iron content: 1280 ppm
> Cobalt content: 92 ppm
> Alumina content: 0.05% by mass
> Zirconia content: remainder

The prepared powder composition had a BET specific surface area of 10.1 $m^2/g$, an average particle size of 0.45 $\mu$m, a crystallite size of 370 nm, a monoclinic ratio of 7% and an average granule size of 45 pm.

COMPARATIVE EXAMPLE 3

(Terbium Oxide Granular Powder)

The yttrium-stabilized zirconia powder prepared in Comparative Example 1 was partially taken and mixed with an $\alpha$-alumina powder, a terbium oxide powder and pure water in a ball mill to prepare a slurry. An acrylic acid binder was added to and mixed with the slurry such that the mass ratio of the binder to the powder in the slurry was 3% by mass. The slurry was spray-dried in air at 180°C to prepare a granular powder (hereinafter also referred to as a "Tb-Y(4.3)-stabilized $ZrO_2$ granular powder") containing 3% by mass of the acrylic acid binder, 0.05% by mass of alumina and 0.04% by mole (0.24% by mass) of terbium oxide with the remainder being zirconia stabilized by 4.3% by mole of yttrium. The granular powder had an average granule size of 45 $\mu$m and contained terbium in the form of an oxide, and the terbium was not dissolved in zirconia.

(Granular Powder Containing Cobalt Oxide and Titanium Oxide)

A cobalt oxide powder ($Co_3O_4$) and a titanium oxide powder ($TiO_2$) were used as coloring metal powders. The yttrium-stabilized zirconia powder prepared in Comparative Example 1 was partially taken and mixed with an $\alpha$-alumina powder, a cobalt oxide powder, a titanium oxide powder and pure water in a ball mill to prepare a slurry. An acrylic acid binder was added to and mixed with the slurry such that the mass ratio of the binder to the powder in the slurry was 3% by mass. The slurry was spray-dried in air at 180°C to prepare a granular powder (hereinafter also referred to as a "Co-Ti-Y(4.3)-stabilized $ZrO_2$ granular powder") containing 3% by mass of the acrylic acid binder, 0.05% by mass of alumina, 0.06% by mass of tricobalt tetraoxide and 0.30% by mass of titanium oxide with the remainder being zirconia stabilized by 4.3% by mole of yttrium. The granular powder had an average granule size of 45 pm.

(Powder Composition)

A powder composition according to the present comparative example with the following composition and with a coloring metal element content of 144 ppm was prepared in the same manner as in Example 1 except that the Y(4.3)-stabilized $ZrO_2$ granular powder, the Er(4.4) solid solution $ZrO_2$ granular powder, the Tb-Y(4.3)-stabilized $ZrO_2$ granular powder and the Co-Ti-Y(4.3)-stabilized $ZrO_2$ granular powder were mixed at a mass ratio of 85.6:3.5:6.4:4.5.

Yttrium content: 4.154% by mole
Erbium content: 0.146% by mole
Terbium content: 0.003% by mole (0.02% by mass)
Cobalt content: 24 ppm
Titanium content: 120 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 angstroms, a monoclinic ratio of 7% and an average granule size of 44 $\mu$m.

Comparative Example 4

A powder composition according to the present comparative example with the following composition and with a coloring metal element content of 630 ppm was prepared in the same manner as in Comparative Example 1 except that the Y(4.3)-stabilized $ZrO_2$ granular powder, the Er(4.4) solid solution $ZrO_2$ granular powder, the Tb-Y(4.3)-stabilized $ZrO_2$ granular powder and the Co-Ti-Y(4.3)-stabilized $ZrO_2$ granular powder were dry-mixed at a mass ratio of 61.2:1.3:20.0:17.5.

Yttrium content: 4.248% by mole
Erbium content: 0.053% by mole
Terbium content: 0.008% by mole (0.05% by mass)
Cobalt content: 105 ppm
Titanium content: 525 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 nm, a monoclinic ratio of 7% and an average granule size of 45 $\mu$m.

Comparative Example 5

A powder composition according to the present comparative example with the following composition and with a coloring metal element content of 900 ppm was prepared in the same manner as in Comparative Example 1 except that the Y(4.3)-stabilized $ZrO_2$ granular powder, the Er(4.4) solid solution $ZrO_2$ granular powder, the Tb-Y(4.3)-stabilized $ZrO_2$ granular powder and the Co-Ti-Y(4.3)-stabilized $ZrO_2$ granular powder were dry-mixed at a mass ratio of 22.5:6.5:46.0:25.0.

Yttrium content: 4.039% by mole
Erbium content: 0.273% by mole
Terbium content: 0.019% by mole (0.11% by mass)
Cobalt content: 150 ppm
Titanium content: 750 ppm
Alumina content: 0.05% by mass
Zirconia content: remainder

The prepared powder composition had a BET specific surface area of 10.1 m$^2$/g, an average particle size of 0.45 $\mu$m, a crystallite size of 370 nm, a monoclinic ratio of 7% and an average granule size of 45 pm.

(Green Body, Calcined Body and Sintered Body)

Green bodies (green compacts), calcined bodies and sintered bodies were prepared in the same manner as in Example 1 except that the powder compositions prepared in Comparative Examples 2 to 5 were used.

The following tables show the results of the powder compositions and the calcined bodies prepared in Examples and Comparative Examples.

[Table 3]

| | Y₂O₃ (mol %) | Er₂O₃ (mol %) | Tb₄O₇ (mol %) | Nd₂O₃ (mol %) | Pr₆O₁ (mol %) | Al₂O 3 (wt %) | Co₃O 4 (PP m) | TiO₂ (pp m) | Fe₂O 3 (pp m) | BET specific surface area (m²/g) | Granule size (pm) |
|---|---|---|---|---|---|---|---|---|---|---|---|
| Example 1 | 4.15 4 | 0.14 6 | 0.00 3 | 0 | 0 | 0.0 5 | 24 | 120 | 0 | 10.1 | 44 |
| Example 2 | 4.03 7 | 0.27 3 | 0.01 9 | 0 | 0 | 0.0 5 | 150 | 750 | 0 | 10.2 | 44 |
| Example 3 | 4.24 4 | 0.05 3 | 0.00 2 | 0 | 0 | 0.0 5 | 15 | 75 | 0 | 10.1 | 44 |
| Example 4 | 4.17 6 | 0.12 5 | 0.00 6 | 0 | 0 | 0.0 5 | 30 | 150 | 0 | 10.1 | 44 |
| Example 5 | 4.08 9 | 0.21 5 | 0.00 9 | 0 | 0 | 0.0 5 | 45 | 225 | 0 | 10.1 | 44 |
| Example 6 | 3.91 7 | 0.39 5 | 0.01 4 | 0 | 0 | 0.0 5 | 120 | 600 | 0 | 10.1 | 44 |
| Example 7 | 4.26 2 | 0.03 5 | 0.00 2 | 0 | 0 | 0.0 5 | 15 | 75 | 0 | 10.1 | 44 |
| Example 8 | 4.22 7 | 0.07 1 | 0.00 3 | 0 | 0 | 0.0 5 | 20 | 98 | 0 | 10.1 | 44 |
| Example 9 | 4.18 2 | 0.11 8 | 0.00 5 | 0 | 0 | 0.0 5 | 38 | 188 | 0 | 10.1 | 44 |
| Example 10 | 4.17 7 | 0.12 5 | 0.01 0 | 0 | 0 | 0.0 5 | 45 | 225 | 0 | 10.1 | 44 |
| Example 11 | 4.24 5 | 0.05 3 | 0.00 2 | 0 | 0 | 0.0 5 | 45 | 225 | 0 | 10.1 | 44 |
| Example 12 | 4.21 1 | 0.08 9 | 0.00 4 | 0 | 0 | 0.0 5 | 68 | 338 | 0 | 10.1 | 44 |
| Example 13 | 4.24 8 | 0.05 3 | 0.00 8 | 0 | 0 | 0.0 5 | 105 | 525 | 0 | 10.1 | 44 |
| Example 14 | 4.17 7 | 0.12 5 | 0.00 6 | 0 | 0 | 0.0 5 | 75 | 375 | 0 | 10.1 | 44 |
| Example 15 | 4.08 9 | 0.21 5 | 0.00 9 | 0 | 0 | 0.0 5 | 53 | 263 | 0 | 10.1 | 44 |
| Example 16 | 4.22 9 | 0.07 1 | 0.00 7 | 0 | 0 | 0.0 5 | 56 | 278 | 0 | 10.1 | 44 |
| Example 17 | 3.02 5 | 0.08 3 | 0.00 3 | 0 | 0 | 0.0 5 | 20 | 98 | 0 | 12.6 | 44 |
| Example 18 | 3.41 0 | 0.05 3 | 0.00 8 | 0 | 0 | 0.0 5 | 105 | 525 | 0 | 11.8 | 44 |
| Example 19 | 3.84 4 | 0.17 8 | 0.01 8 | 0 | 0 | 0.0 5 | 160 | 800 | 0 | 10.6 | 44 |
| Example 20 | 3.02 5 | 0.08 3 | 0.00 3 | 0 | 0 | 0.0 5 | 20 | 98 | 0 | 10.0 | 44 |
| Example 21 | 3.41 0 | 0.05 3 | 0.00 8 | 0 | 0 | 0.0 5 | 105 | 525 | 0 | 10.1 | 44 |
| Example 22 | 3.84 4 | 0.17 8 | 0.01 8 | 0 | 0 | 0.0 5 | 160 | 800 | 0 | 10.1 | 44 |
| Example 23 | 4.94 5 | 0.13 0 | 0.00 7 | 0 | 0 | 0.0 5 | 33 | 165 | 0 | 10.1 | 44 |
| Example 24 | 4.90 5 | 0.02 6 | 0.00 8 | 0 | 0 | 0.0 5 | 108 | 540 | 0 | 10.1 | 44 |
| Example 25 | 4.57 7 | 0.09 3 | 0.01 5 | 0 | 0 | 0.0 5 | 156 | 780 | 0 | 10.1 | 44 |

(continued)

| | Y$_2$O$_3$ (mol %) | Er$_2$O$_3$ (mol %) | Tb$_4$O$_7$ (mol %) | Nd$_2$O$_3$ (mol %) | Pr$_6$O$_{11}$ (mol %) | Al$_2$O$_3$ (wt %) | Co$_3$O$_4$ (PP m) | TiO$_2$ (pp m) | Fe$_2$O$_3$ (pp m) | BET specific surface area (m2/g) | Granule size (pm) |
|---|---|---|---|---|---|---|---|---|---|---|---|
| Example 26 | 4.127 | 0.132 | 0.003 | 0.023 | 0 | 0.05 | 0 | 15 | 0 | 10.1 | 44 |
| Example 27 | 3.667 | 0.135 | 0.016 | 0.288 | 0 | 0.05 | 0 | 189 | 0 | 10.1 | 44 |
| Example 28 | 3.320 | 0.126 | 0.024 | 0.492 | 0 | 0.05 | 0 | 323 | 0 | 10.1 | 44 |
| Example 29 | 4.225 | 0.042 | 0 | 0 | 0.012 | 0.05 | 92 | 458 | 0 | 10.1 | 44 |
| Example 30 | 4.210 | 0.036 | 0 | 0 | 0.020 | 0.05 | 92 | 458 | 0 | 10.1 | 44 |
| Example 31 | 4.173 | 0.050 | 0 | 0 | 0.030 | 0.05 | 229 | 850 | 0 | 10.1 | 44 |
| Comparative example 1 | 4.166 | 0.126 | 0 | 0 | 0 | 0.05 | 0 | 0 | 700 | 10.1 | 45 |
| Comparative example 2 | 4.257 | 0.042 | 0 | 0 | 0 | 0.05 | 92 | 0 | 1280 | 10.1 | 45 |
| Comparative example 3 | 4.154 | 0.146 | 0.003 | 0 | 0 | 0.05 | 24 | 120 | 0 | 10.1 | 44 |
| Comparative example 4 | 4.248 | 0.053 | 0.008 | 0 | 0 | 0.05 | 105 | 525 | 0 | 10.1 | 45 |
| Comparative example 5 | 4.039 | 0.273 | 0.019 | 0 | 0 | 0.05 | 150 | 750 | 0 | 10.1 | 45 |

[Table 4]

| | Calcined body | | | |
|---|---|---|---|---|
| | Measured density [g/cm$^3$] | Vickers hardness [HV] | Hardness difference (A2/C4) | Hardness difference (A1) |
| Example 1 | 3.24 | 41 | 5 | 0 |
| Example 2 | 3.24 | 46 | | 5 |
| Example 3 | 3.24 | 41 | | - |
| Example 4 | 3.24 | 44 | | 3 |
| Example 5 | 3.26 | 45 | | 4 |
| Example 6 | 3.26 | 45 | | 4 |
| Example 7 | 3.25 | 44 | | 3 |
| Example 8 | 3.24 | 45 | | 4 |
| Example 9 | 3.25 | 45 | | 4 |
| Example 10 | 3.26 | 45 | | 4 |
| Example 11 | 3.24 | 44 | | 3 |
| Example 12 | 3.24 | 44 | | 3 |
| Example 13 | 3.25 | 45 | | 4 |
| Example 14 | 3.24 | 45 | | 4 |
| Example 15 | 3.25 | 45 | | 4 |
| Example 16 | 3.24 | 45 | | 4 |
| Example 17 | 3.25 | 50 | | 9 |
| Example 18 | 3.24 | 46 | | 5 |
| Example 19 | 3.24 | 40 | | 1 |
| Example 20 | 3.24 | 46 | | 5 |
| Example 21 | 3.24 | 42 | | 1 |
| Example 22 | 3.24 | 40 | | 1 |
| Example 23 | 3.24 | 45 | | 4 |
| Example 24 | 3.23 | 46 | | 5 |
| Example 25 | 3.23 | 46 | | 5 |
| Example 26 | 3.23 | 50 | | 5 |
| Example 27 | 3.23 | 47 | | 3 |
| Example 28 | 3.23 | 46 | | 3 |
| Example 29 | 3.23 | 50 | | 5 |
| Example 30 | 3.23 | 51 | | 5 |
| Example 31 | 3.23 | 51 | | 5 |
| Comparative example 1 | 3.24 | 53 | 14 | 12 |
| Comparative example 2 | 3.26 | 67 | | 26 |
| Comparative example 3 | 3.24 | 57 | | 16 |

(continued)

| | Calcined body | | | |
|---|---|---|---|---|
| | Measured density [g/cm³] | Vickers hardness [HV] | Hardness difference (A2/C4) | Hardness difference (A1) |
| Comparative example 4 | 3.25 | 61 | | 20 |
| Comparative example 5 | 3.26 | 72 | | 31 |

The calcined bodies according to Examples and Comparative Examples were prepared under the same conditions except that the powder compositions used were different. All the calcined bodies had Vickers hardness suitable for CAD/CAM processing. However, Examples 1 (color tone: A2) and 2 (color tone: C4) have a hardness difference (A2/C4) of 5, whereas Comparative Examples 1 (color tone: A2) and 2 (color tone: C4) have a hardness difference (A2/C4) of 14, indicating that the Comparative Examples have greatly different processability due to the color tone difference.

Examples 1, 2 and 4 to 16 have a hardness difference (A1) of 5 or less and have similar processability regardless of the color tone difference. Examples 17 to 31 have a hardness difference (A1) of 9 or less and have similar processability regardless of the color tone difference. By contrast, Comparative Examples 1 and 2 have a hardness difference (A1) of 12 and 26, respectively, and have different processability due to the color tone difference. Comparative Examples 3 to 5 have a large hardness difference (A1) and have different processability due to the color tone difference.

The following table shows the characteristics of the sintered bodies prepared in Examples and Comparative Examples.

[Table 5]

| | Sintered body | | | | |
|---|---|---|---|---|---|
| | Color tone | | | Bending strength (MPa) | Total light transmittance (%) |
| | L* | a* | b* | | |
| Example 1 | 87.7 | 2.5 | 16.5 | 1063 | 39 |
| Example 2 | 73.4 | 5.2 | 19.2 | 1035 | 26 |
| Example 3 | 89.9 | 0.2 | 13.9 | - | 41 |
| Example 4 | 85.3 | 3.1 | 23.5 | - | 37 |
| Example 5 | 82.6 | 5.2 | 25.0 | - | 35 |
| Example 6 | 75.3 | 7.2 | 20.6 | - | 28 |
| Example 7 | 90.0 | -0.6 | 17.0 | - | 41 |
| Example 8 | 88.0 | 1.1 | 19.0 | - | 39 |
| Example 9 | 85.1 | 2.7 | 20.1 | - | 36 |
| Example 10 | 83.4 | 3.9 | 27.0 | - | 35 |
| Example 11 | 86.0 | 1.3 | 11.4 | - | 37 |
| Example 12 | 82.3 | 2.6 | 16.6 | - | 34 |
| Example 13 | 78.9 | 2.6 | 18.7 | - | 30 |
| Example 14 | 80.8 | 3.6 | 18.2 | - | 33 |
| Example 15 | 81.5 | 5.6 | 24.0 | - | 34 |
| Example 16 | 82.8 | 2.6 | 22.3 | - | 35 |
| Example 17 | 89.3 | 1.5 | 16.4 | - | 37 |
| Example 18 | 79.0 | 2.6 | 17.7 | - | 28 |

(continued)

| | Sintered body | | | | |
|---|---|---|---|---|---|
| | Color tone | | | Bending strength (MPa) | Total light transmittance (%) |
| | L* | a* | b* | | |
| Example 19 | 73.3 | 5.1 | 18.7 | - | 24 |
| Example 20 | 89.3 | 1.5 | 16.4 | - | 37 |
| Example 21 | 78.3 | 2.8 | 18.2 | - | 28 |
| Example 22 | 73.1 | 5.4 | 19.4 | - | 24 |
| Example 23 | 84.0 | 1.9 | 21.2 | - | 41 |
| Example 24 | 79.4 | 0.9 | 18.2 | - | 35 |
| Example 25 | 75.0 | 3.7 | 19.9 | - | 29 |
| Example 26 | 89.5 | 1.9 | 16.5 | - | 40 |
| Example 27 | 78.1 | 3.5 | 20.2 | - | 27 |
| Example 28 | 75.0 | 4.9 | 18.1 | - | 21 |
| Example 29 | 86.2 | 1.8 | 15.8 | - | 32 |
| Example 30 | 79.7 | 1.74 | 20.1 | - | 32 |
| Example 31 | 73.5 | 3.6 | 19.7 | - | 23 |
| Comparative example 1 | 88.2 | 1.5 | 19.2 | 1069 | 39 |
| Comparative example 2 | 73.1 | 4.9 | 21.5 | 1081 | 26 |
| Comparative example 3 | 88.5 | 2.2 | 15.1 | - | 39 |
| Comparative example 4 | 79.7 | 2.0 | 17.6 | - | 31 |
| Comparative example 5 | 73.4 | 4.7 | 18.8 | - | 25 |

The table shows that both Example 1 and Comparative Example 1 have a color tone corresponding to A2, and both Example 2 and Comparative Example 2 have a color tone corresponding to C4. Furthermore, these have a bending strength of 1000 MPa or more and have mechanical strength suitable for a dental prosthetic material.

Furthermore, Examples 3 to 16 have the color tone of A1, A3, A3.5, A4, B1, B2, B3, B4, C1, C2, C3, D2, D3 or D4 in the Vita classical shade. Examples 17 to 31 have the color tone of A2, B3, C3 or C4 in the Vita classical shade.

The entire contents of the description, claims and abstract of Japanese Patent Application No. 2021-9019 filed on January 22, 2021 and Japanese Patent Application No. 2021-183423 filed on November 10, 2021 are hereby incorporated by reference as the disclosure of the description of the present disclosure.

**Claims**

1. A powder composition comprising: two or more types of zirconia in which a lanthanoid rare-earth element is dissolved; a transition metal element other than zirconium and hafnium; and a remainder composed of zirconia stabilized only by one or more selected from the group consisting of yttrium, calcium and magnesium, wherein a different lanthanoid rare-earth element is dissolved in each zirconia in which the lanthanoid rare-earth element is dissolved, and a transition metal element content is 1500 ppm or less.

2. The powder composition according to Claim 1, wherein at least one type of the zirconia in which the lanthanoid rare-earth element is dissolved is zirconia in which one or more selected from the group consisting of praseodymium, samarium, terbium, dysprosium, holmium and thulium are dissolved.

3. The powder composition according to Claim 1 or 2, wherein at least one type of the zirconia in which the lanthanoid rare-earth element is dissolved is zirconia in which one or more selected from the group consisting of neodymium and erbium are dissolved.

4. The powder composition according to any one of Claims 1 to 3, wherein at least one type of the zirconia in which the lanthanoid rare-earth element is dissolved is zirconia stabilized by one or more selected from the group consisting of yttrium, calcium and magnesium.

5. The powder composition according to any one of Claims 1 to 4, wherein the transition metal element is one or more selected from the group consisting of manganese, cobalt and titanium.

6. The powder composition according to any one of Claims 1 to 5, wherein the transition metal element is contained as one or more selected from the group consisting of oxides, hydroxides, oxyhydroxides, chlorides, sulfates and nitrates.

7. The powder composition according to any one of Claims 1 to 6, wherein the remainder is zirconia stabilized only by yttrium.

8. The powder composition according to any one of Claims 1 to 7, wherein an iron content is 100 ppm or less.

9. The powder composition according to any one of Claims 1 to 8, comprising alumina.

10. The powder composition according to any one of Claims 1 to 9, comprising a granular particle composed of the transition metal element other than zirconium and hafnium and the zirconia stabilized only by one or more selected from the group consisting of yttrium, calcium and magnesium.

11. The powder composition according to any one of Claims 1 to 10, wherein a BET specific surface area is 5 $m^2$/g or more and 15 $m^2$/g or less.

12. A method for producing a calcined body using the powder composition according to any one of Claims 1 to 11.

13. A method for producing a sintered body using the powder composition according to any one of Claims 1 to 11.

14. A calcined body comprising a fused particle of a transition metal compound other than zirconium and hafnium, zirconia in which two or more lanthanoid rare-earth elements are dissolved, and zirconia stabilized only by one or more selected from the group consisting of yttrium, calcium and magnesium, wherein the transition metal element other than zirconium and hafnium constitutes 1500 ppm or less.

15. A method for producing a sintered body, comprising using the calcined body according to Claim 14.

## INTERNATIONAL SEARCH REPORT

| | International application No. |
|---|---|
| | **PCT/JP2022/001817** |

**A.    CLASSIFICATION OF SUBJECT MATTER**

*C01G 25/02*(2006.01)i; *A61C 5/70*(2017.01)i; *A61C 13/083*(2006.01)i; *A61K 6/818*(2020.01)i; *A61K 6/822*(2020.01)i; *A61K 6/824*(2020.01)i; *C01G 25/00*(2006.01)i; *C04B 35/486*(2006.01)i; *C04B 35/488*(2006.01)i
FI:   C01G25/02; A61C5/70; A61C13/083; A61K6/818; A61K6/822; A61K6/824; C01G25/00; C04B35/486; C04B35/488

According to International Patent Classification (IPC) or to both national classification and IPC

**B.    FIELDS SEARCHED**

Minimum documentation searched (classification system followed by classification symbols)

C01G25/02; A61C5/70; A61C13/083; A61K6/818; A61K6/822; A61K6/824; C01G25/00; C04B35/486; C04B35/488

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 2020-147495 A (TOSOH CORP.) 17 September 2020 (2020-09-17) | 1-7, 9-10, 12-15 |
| | claims 1-14, paragraphs [0041], [0053]-[0056], [0139]-[0149] | |
| Y | claims 1-14, paragraphs [0041], [0053]-[0056], [0139]-[0149] | 11 |
| A | | 8 |
| Y | JP 2020-132518 A (TOSOH CORP.) 31 August 2020 (2020-08-31) | 11 |
| | paragraphs [0041], [0062] | |
| A | | 1-10, 12-15 |
| Y | WO 2018/155459 A1 (SHOFU INC.) 30 August 2018 (2018-08-30) | 11 |
| | claims 1-26,paragraph [0141] | |
| A | | 1-10, 12-15 |
| A | WO 2020/218541 A1 (KURARAY NORITAKE DENTAL INC.) 29 October 2020 (2020-10-29) | 1-15 |
| | entire text, all drawings | |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 March 2022** | **05 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| | International application No. |
|---|---|
| | **PCT/JP2022/001817** |

**C.    DOCUMENTS CONSIDERED TO BE RELEVANT**

| Category* | Citation of document, with indication, where appropriate, of the relevant passages | Relevant to claim No. |
|---|---|---|
| A | US 2018/0235847 A1 (GLIDEWELL JAMES R DENTAL CERAMICS INC.) 23 August 2018 (2018-08-23)<br>       entire text, all drawings | 1-15 |

Form PCT/ISA/210 (second sheet) (January 2015)

**INTERNATIONAL SEARCH REPORT**
Information on patent family members

International application No.

**PCT/JP2022/001817**

| Patent document cited in search report | | | Publication date (day/month/year) | Patent family member(s) | Publication date (day/month/year) |
|---|---|---|---|---|---|
| JP | 2020-147495 | A | 17 September 2020 | US 2020/0283341 A1<br>claims 1-20, paragraphs [0078], [0090]-[0093], [0219]-[0231]<br>EP 3705081 A1<br>KR 10-2020-0108249 A<br>CN 111658552 A | |
| JP | 2020-132518 | A | 31 August 2020 | US 2020/0262759 A1<br>paragraphs [0126], [0166] | |
| WO | 2018/155459 | A1 | 30 August 2018 | US 2020/0113658 A1<br>claims 1-26, paragraph [0144]<br>EP 3611150 A1<br>KR 10-2019-0122223 A<br>CN 110678435 A | |
| WO | 2020/218541 | A1 | 29 October 2020 | KR 10-2022-0002353 A<br>CN 113710627 A | |
| US | 2018/0235847 | A1 | 23 August 2018 | DE 102018103906 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014022643 A
- US 9962247 B
- WO 2016019114 A
- JP 2021009019 A
- JP 2021183423 A